(19) Europäisches Patentamt European Patent Office Office européen des brevets

(11) **EP 0 581 871 B2**

(12) **NEW EUROPEAN PATENT SPECIFICATION**
After opposition procedure

(45) Date of publication and mention of the opposition decision:
**12.08.2009 Bulletin 2009/33**

(45) Mention of the grant of the patent:
**16.12.1998 Bulletin 1998/51**

(21) Application number: **92911842.0**

(22) Date of filing: **29.04.1992**

(51) Int Cl.:
**G01B 9/02** (2006.01)

(86) International application number:
**PCT/US1992/003536**

(87) International publication number:
**WO 1992/019930 (12.11.1992 Gazette 1992/28)**

(54) **APPARATUS FOR OPTICAL IMAGING AND MEASUREMENT**

VORRICHTUNG FÜR OPTISCHE ABBILDUNG UND MESSUNG

APPAREIL D'IMAGERIE OPTIQUE ET DE MESURE

(84) Designated Contracting States:
**CH DE FR GB IT LI SE**

(30) Priority: **29.04.1991 US 692877**

(43) Date of publication of application:
**09.02.1994 Bulletin 1994/06**

(73) Proprietors:
• **MASSACHUSETTS INSTITUTE OF TECHNOLOGY**
**Cambridge, MA 02139 (US)**
• **SWANSON, Eric, A.**
**Maynard, MA 01754 (US)**

(72) Inventors:
• **HUANG, David**
**Cambridge, MA 02139 (US)**
• **FUJIMOTO, James, G.**
**Cambridge, MA 02139 (US)**
• **PULIAFITO, Carmen, A.**
**Weston, MA 02193 (US)**
• **LIN, Charles, P.**
**Somerville, MA 02144 (US)**
• **SCHUMAN, Joseph, S.**
**Boston, MA 02114 (US)**

(74) Representative: **Geyer, Fehners & Partner Patentanwälte**
**Perhamerstraße 31**
**80687 München (DE)**

(56) References cited:
**EP-A- 0 484 913        DE-A1- 2 528 209**
**DE-A1- 3 201 801        GB-A- 2 191 855**
**US-A- 4 627 731        US-A- 4 928 005**

• **OPTICAL FIBER SENSORS - TECHNICAL DIGEST SERIES vol. 2, no. 1 , 27 January 1988 , WASHINGTON (US) pages 56 - 58 A.S. GERGES E.A. 'A SHORT COHERENCE LENGTH INTERFEROMETRIC FIBRE OPTIC SENSOR SYSTEM'**
• **P.G. SUCHOSKI E.A.: 'MINIATURE LASER VIBROMETER SYSTEM WITH MULTIFUNCTION INTEGRATED OPTIC CIRCUIT' IEEE PHOTONICS TECHNOLOGY LETTERS vol. 2, no. 1, January 1990, NEW YORK US, pages 81 - 82, XP000114146**
• **M. DAVIDSON E.A.: 'AN APPLICATION OF INTERFERENCE MICROSCOPY TO INTEGRATED INSPECTION AND METROLOGY' PROCEEDINGS OF SPIE vol. 775, 04 March 1987, WASHINGTON (US), pages 233 - 247, XP000923197**
• **OPTIC LETTERS, vol. 12, no. 3, Mrach 1987, pages 158 - 160; R.C. Youngquist et al.:" Optical Coherence -Domain Reflectometry: a new Optical Evaluation Technique"**
• **APPLIED OPTICS, vol. 29, no. 26; 10.September 1990, pages 3784 - 3788; B.S.Lee et al.:"Profilometry with a Coherence Scanning Microscope"**
• **APPLIED OPTICS, vol. 27, no. 27, 15. January 1988, pages 306 - 311; A.J. de Boef: "Two-Wavelength Scanning Spot Interferometer Using Single-Frequency Diode Lasers"**
• **INVESTIGATIVE OPHTHALMOLOGY AND VISUAL SCIENCE, vol. 32, no. 3, March 1991, pages 616 - 624; Hitzenberger: "Optical Measurement of the Axial EyE Length by Laser Doppler Interferometry"**
• **SCIENCE, vol. 254, 22. Novembre 1991, pages 1178 - 1181; D. Huang et al.: "Optical Coherence Tomography"**

- **A.J. DEN BOEF: 'Interferometric Laser Rangefinder Using a Frequency Modulated' APPLIED OPTICS vol. 26, 01 November 1987, pages 4545 - 4550**
- **M. KOBAYASHI ET AL.: 'Polarization-independent Interferometric Optical-Time-Domain' JOURNAL OF LIGHTWAVE TECHNOLOGY vol. 9, no. 5, 05 May 1991, pages 623 - 628**
- **W.V SORIN: 'Simultaneous thickness and Group Index Measurement Using' IEEE PHOTONIC TECHNOLOGY LETTERS vol. 4, no. 1, 01 January 1992, pages 105 - 107**

**Description**

Field of the Invention

This invention relates to optical imaging, including utilizing such images to perform precision measurements on biological and other samples.

Background of the Invention

There are many industrial, medical, and other applications where high resolution (generally less than 10 micrometer) images of, and measurements of distances, thicknesses, and optical properties of, a biological or other sample are required.

Existing techniques for performing such measurements include optical coherence domain reflectometers (OCDR), optical time domain reflectometry (OTDR), ultrasound, scanning laser microscopes, scanning confocal microscopes, scanning laser ophthalmoscopes and optical triangulation. Existing OCDR systems do not normally have the rapid data acquisition rate required for the measurement of biological or other samples having the potential for dynamic movement; while OTDR systems are very expensive and have only limited resolution and dynamic range.

Ultrasound, which is perhaps the most commonly used technique, is disadvantageous for applications such as taking measurements on the eye in that, in order to achieve the required acoustic impedence matches, and to thus avoid beam losses and distortion, contact is generally required between the ultrasonic head or probe and the product or patient being scanned. While such contact is not a problem when scans are being performed on, for example, a patient's chest, such probes can cause severe discomfort to a patient when used for taking eye measurements such as those used for measuring intraocular distances for computing the power of lens implants.

The relatively long wavelengths employed in ultrasound also limit spatial resolution. Further, ultrasound depends on varying ultrasound reflection and absorption characteristics to differentiate and permit recording or display of tissue, or other boundaries of interest. Therefore, when the acoustic characteristics of adjacent layers to be measured are not significantly different, ultrasound may have difficulty recognizing such boundaries.

Scanning laser or confocal microscopes and scanning laser ophthalmoscopes (SLO) provide highly spatially resolved images, for example being able to gnerate real time video images of the eye with a lateral resolution of a few micrometers. However, the depth resolution of SLO's quickly degrade with decreasing numerical aperture. For example, SLO measurements of the retina through the pupil aperture restrict the depth resolution to roughly 200 microns. SLO's are also expensive, costing in the range of a quarter million dollars.

Optical triangulation offers fairly high resolution, but requires parallel boundaries. Such devices also have relatively poor signal-to-noise ratios and have degraded resolution at greater depths, where numerical aperture is restricted.

In DE-A1-2528209 an interferometer for surface profilometry is disclosed, with which the object to be measured is arranged in the measurement beam path of the interferometer and a reference reflector is arranged in a reference beam path. The reference reflector is periodically moved along the direction of the reference beam path and the position of the reference mirror in which interference occurs between light reflected at the reference reflector and light reflected at the object surface is a measure for the position of the object surface in the direction of the measuring beam path.

This known apparatus does not provide the possibility to perform high resolution measurements of distances or thicknesses between or of sample layers of for example biological samples such as an eye.

DE-A1-3201801 discloses an apparatus for measuring distances in a living eye. The eye is illuminated and the light reflected at different layers of the eye is visually observed through an interferometer with which the length of one beam path can be changed. Interference between light reflected at different layers occurs if the path length difference within the eye is compensated by a respective path length difference within the interferometer.

However, if the distances of different layers within the eye are the same or nearly the same such layers cannot be distinguished since the respective interferences occur at the same path length difference within the interferometer.

GB-A-21 91 855 describes a method and apparatus for detecting reflection sites within a sample by vertically scanning through the sample. The reflection sites within the sample are detected by directing light towards the sample and a reflecting surface, moving either the reflecting surface or the sample to vary the depth within the sample from which reflection and thus interference patterns can be determined and detecting the interference from the combined light beams reflected within the sample and on the reflecting surface.

An Optical coherence tomograph allowing cross sectional imaging of biological systems is described in Huang et al., "Optical coherence tomography", Science, Vol. 254, 22 November 1991, p.1178-1181. An scanning interferometer which utilizes two-wavelengths is disclosed in den Boef, "Two-wavelength scanning spot interferometer using single-frequency diode Lasers", Applied Optics, Vol. 27, No. 2, 15 January 1998, p. 306-309.

A need, therefore, exists for an improved method and apparatus for performing high resolution measurements and in particular for optically performing such measurements, which improved technique does not require contact with the body being measured, which maintains substantially constant high resolution over a scanning depth of interest, regardless of available aperture size and which is relatively compact and inexpensive to manufacture. Such a system should also be capable of providing differentiation between sample layers, should be able to provide identification of layer material or of selected properties thereof, should be able to provide one, two and three-dimensional images of a scanned body and should be rapid enough for use in biological and other applications where the sample being measured changes over relatively short time intervals. Finally, it would be desirable if such technique could also provide information concerning the birefringence property and spectral properties of the sample.

More specifically, a need exists for a means to perform scanning of a sample in at least one transverse direction at a selected longitudinal depth, with the capability of also scanning in the longitudinal direction. Further, particularly in medical application, it is frequently desirable to provide such scans inside of tubular or other structures such as blood vessels, the bronchial tree of the lungs, the gastrointestinal tract, the genital tract or the urinary tract, using an angioscope or endoscope. In order for such scanning to be performed, a probe must be provided which is capable of being mounted in an endoscope or angioscope for performing internal scans.

While typically a scan would be completed through the full depth range at a given lateral and/or transverse position before repositioning to the next position, this may require scanning of the mirror or other element used for performing longitudinal range or depth scans at a rate higher than the capacity of existing equipment. This is particularly true where the longitudinal scan produces a Doppler shift frequency which affects the interferometric signal frequency and hence the system sensitivity. It is, therefore, desired that such scanning be performed at a constant velocity. However, since very high speed longitudinal scanning at a constant velocity is difficult to achieve, where two or three dimensional scanning is being performed, other scan patterns may be required. Further, in some applications, it may be desirable to perform transverse scanning in one or two dimensions at a selected longitudinal position or depth.

Another problem which becomes particularly serious when transverse scanning is being performed is that the bandwidth of the received signals increases beyond the inherent Doppler frequency shift of the system. In such cases, aliasing (i.e. variations in image intensity) may occur. It is, therefore, desirable that a technique be provided to enhance resolution by eliminating or averaging out such intensity variations.

Another problem with the prior system is that, if scanning is to be conducted over an extended depth range, a smaller numerical aperture must be used so as to extend the depth of focus. However, this reduces lateral resolution and the received optical signal power throughout the range. A need, therefore, exists for a technique which permits the use of a large numerical aperture over an extended depth range within a sample.

Further, some of the problems described which result from performing longitudinal scanning by mechanically moving a mirror or other element may be overcome by performing this scan electronically, for example by varying the optical frequency or amplitude of the light incident from the light source. However, for certain applications, for example imaging a dynamic biological sample such as the eye, the scanning speed required to do three-dimensional scanning may be such that a parallel scanning technique may be preferable or may be required.

Thus, a need exists for improved optical coherence domain reflectometer (OCDR) optical imaging and measurement systems or for other imaging and measurement systems, particularly electronically scanned systems, which are capable of performing one, two and three dimensional scans and measurements at a selected and/or over an extended longitudinal or depth range for either internal or external samples with sharp focus and high resolution and sensitivity over the range.

At least some of the above problems are solved according to the invention by an apparatus comprising the features of claim 1 or 13. Further advantageous embodiments of the invention comprise the features of the dependent claims.

In accordance with the above, this invention provides a method and apparatus for performing optical imaging and measurements on a sample by applying optical radiation, which radiation has a short coherence length for preferred embodiments, to a reference optical reflector and to the sample through first and second optical paths respectively. The optical paths are preferably fiber optic paths. The longitudinal range within the sample from which imaging information is obtained is controlled by, for example, altering the relative lengths of the paths or by varying the frequency or intensity of the source in accordance with a predetermined profile. The lateral or transverse position on the sample at which imaging or measurements are being performed may also be selectively changed. This can result in imaging being performed on the sample in at least one transverse dimension. Where the profile for longitudinal scanning is a steppable profile, transverse scanning in one or two dimensions may be performed at any selected longitudinal range. Reflections from the reflector through the first optical path and reflections from the sample received through the second optical path are combined, the resulting combined optical output having interference fringes at matched points, for example, length matched points, on the two paths and having an instantaneous modulating frequency which may include a Doppler shift frequency at a frequency $f_D \sim NV/\lambda$ for embodiments where relative path lengths are being altered with a velocity profile having an instantaneous velocity V at each point on the profile. The combined output is detected and the detected output is processed to obtain a selected image of the sample and/or information concerning selected measurements.

For embodiments where V is to be substantially constant over the scan range, the changes in first path length may be ramped, with the change in one direction occurring at the velocity V and the change in the other direction occurring much more rapidly. The changes in first path length may also have a triangular pattern, with the change in at least one direction being at the velocity V. The scan pattern may also be a sinusoidal pattern. With uniform velocity, measurements would be taken during a translation which occurs at the velocity V and may occur for path length changes in both directions with a triangular drive. With a sinusoidal drive, the nonlinearity may be detected and taken into account in subsequent processing.

The system is preferably implemented utilizing optical fibers in the optical paths; however, the system may also be implemented utilizing bulk optics or other optical components. Where optical fibers are employed, the lengths of the paths and the lengths of the fibers in the paths are preferably both substantially equal.

The changes in the first optical path are preferably accomplished by reciprocating the mirror or other reference reflector in a direction substantially perpendicular to the optical path. A suitable means may be provided for maintaining the reflector in alignment in spite of movement and wobble of the reflector as it is moved. The numerical aperture for the coupling to the sample should also correspond to a depth field equal to a predetermined depth extent within the sample over which measurements are to be taken.

For measurements on at least one birefringent layer, the system includes a means for polarizing the optical energy from the source in a selected first direction, the polarization of the light being altered differently for energy applied to the reflector and to the sample. The elements which alter the polarization also cause reflected light energy from the reflector to be polarized in a second selected direction and cause reflected light energy from the sample to be polarized in a direction dependent on the birefrigence of the birefringent sample. The combined outputs containing interferometric fringes are split and detected as two outputs having orthogonal polarizations. These two outputs are then separately processed to obtain separate interferometric signals and the separate interferometric signals are combined to provide selected indications of birefringence.

In order to enhance the ability of the system to distinguish a boundary between layers having similar optical properties, and to obtain other information concerning such layers, advantage is taken of the fact that the optical absorption, impedance, and other optical characteristics of materials may vary with wavelength. Thus, one layer of a junction may be more easily detected at a first wavelength of the optical energy, while another layer may be more easily detected at a different wavelength. For one embodiment of the invention, two or more short coherence length optical sources provide optical radiation at different wavelengths, for example $\lambda 1$ and $\lambda 2$, the sample reacting differently to inputs received at these different wavelengths. This results in a first interferometric optical output modulated at a frequency $f_{D1}=2V/\lambda_1$ and a second interferometrical optical output modulated at a frequency $f_{D2}=2V/\lambda_2$. The two outputs are separately demodulated and may then be separately processed or processed together.

The second optical path is terminated in a probe module which preferably includes a means for controlling the transverse position on the sample at which imaging and/or measurement are being performed, and a means for selectively changing this position in at least one dimension to scan the sample. The velocity V may be sufficiently high so that the Doppler shift frequency is sufficiently high to meet the bandwidth requirements to overcome the predominate low frequency noise for the system and for signal aliasing. Where this is not the case, a means is provided for causing a vibratory or other change in a modulating frequency $f_M$, resulting in a modulating frequency which is a selected combination of $f_D$ and $f_M$. This change may be effected by a piezoelectric transducer or by at least one acousto-optic modulator (AOM) in at least one of the optical paths. For one embodiment, there are two AOM's in one of the optical paths, with $f_M$ being the difference frequency shift caused by the two AOM's. The probe position controller may include means for moving a probe at the end of the second optical path or the distal end of a fiber optic element forming the second optical path in at least one dimension substantially perpendicular to the direction in which optical radiation is applied to the sample to provide two-dimensional or three-dimensional scanning of the sample.

For other embodiments, the probe module may include mirrors or other means for steering the optical radiation to a position on the sample and for optically changing the transverse position in at least one dimension, generally perpendicular to the direction in which the optical radiation is applied to the sample. Where three dimensional scanning is desired, the transverse position is changed in two directions. The means for optically changing transverse position may include at least one movable mirror in the optical path of the radiation for angularly translating the radiation at an angle dependent on mirror position. One mirror may be movable in two orthogonal directions to angularly translate the radiation in a direction which varies in two dimensions, whereby three dimensional scanning is achieved, or this objective may be achieved utilizing two mirrors successively spaced along the optical path, with the mirrors being movable in different, generally orthogonal, directions.

For other embodiments, the probe module is a mechanism for scanning internal channels such as an angioscope or endoscope. For such applications, the probe module may include an outer sheath. For one embodiment, the probe module also includes an inner sheath rotatably mounted within the outer sheath, an optical means for directing radiation from the second optical path through the inner sheath, and a means movable with the inner sheath for directing the radiation at a selected position on the internal channel, the selected position varying as the inner sheath is rotated. This embodiment preferably uses a mirror mounted to rotate with the inner sheath to reflect radiation passing through the inner sheath in a selected direction beyond the end of the outer sheath.

For another embodiment, a bundle of optical fibers is mounted in the outer sheath. A first end of a selected one or more of such optical fibers are optically connected to the second optical path, a means being provided for controlling the optical fiber(s) to which the second optical path is connected. There is also a means for establishing a selected transverse position on the sample for each of the optical fibers and for optically connecting a second end of each optical fiber to the corresponding selected transverse position.

For still another embodiment, wherein the first and second optical paths are in the form of first and second optical fibers respectively, the probe includes a means for securing a distal end of the second optical fiber to an inner wall of the sheath. This means includes means for moving the distal end toward and away from the wall. Means are also provided for optically connecting the distal end of the fiber to the sample, this means establishing a selected focal position on the sample for each position of the distal end relative to the wall.

For some embodiments, the probe module includes a means for controlling the focus for the module in the sample so that this depth focus is maintained substantially at a point in the sample from which imaging information is being obtained as this point is periodically changed during a longitudinal scan of the sample. Such focal plane may be accomplished by moving a focusing lens of the probe module in the direction of the radiation passing therethrough to control focus depth.

Multi-dimensional scanning may be accomplished utilizing at least three different scan patterns. For one scan pattern, the rates at which the relative lengths of the optical paths are altered and at which the transverse position on the sample is changed are such that points at all longitudinal ranges of interest are scanned for a given sample transverse position before the scan beam is moved to initiate imaging at a new transverse position. Alternatively, the relative rates at which the longitudinal range altering and sample transverse position changing occur may be such that all imaging positions in at least one transverse dimension are scanned at a given longitudinal range in the sample before the longitudinal range is altered to cause scanning at a new range to be performed. The latter scanning procedure may be desirable where very high speed, uniform velocity longitudinal scanning would be required if the first scanning pattern were utilized. A third scanning pattern is to step the longitudinal position control to a selected longitudinal position and to then perform scanning in one or two dimensions at such longitudinal positions.

For some embodiments, a plurality of optical paths may be provided to permit parallel scanning on the sample. In addition, for some embodiments a characteristic of the optical source, such as its frequency or intensity is controlled or varied to control the longitudinal point in the sample being imaged, the received reflections resulting in an output having a frequency proportional to the optical length of the path to the longitudinal point or plane in the sample being imaged at the time. This output is detected and processed to obtain the image.

Because of aliasing or other problems, spurious intensity variations may occur in an image produced as a result of a two or three dimensional scan. To overcome this problem, AOM's may be used as previously indicated or a plurality of scans may be performed on a sample, with the scans being averaged to compensate for intensity variations.

For preferred embodiments, the measurements involve non-invasive cross-sectional imaging in and measurements on biological specimens. One particular useful application for the invention is in producing cross-sectional images of various eye sections.

The foregoing and other objects, features and advantages of the invention will be apparent from the following more particular description of preferred embodiments of the invention as illustrated in the accompanying drawings.

<u>In the Drawings</u>

FIG. 1A is a schematic block diagram of an optical coherence domain reflectometer in accordance with a preferred embodiment of the invention.

FIG. 1B is a schematic block diagram of an alternative embodiment of the invention utilizing a frequency modulated optical source.

FIG. 1C is a schematic block diagram of another fiber optic embodiment of the invention.

FIG. 1D is a schematic block diagram of a bulk optic embodiment of the invention illustrating the use of two separate wavelengths to enhance resolution.

FIG. 2A is a diagram of the envelope of a scan output which might be obtained utilizing the embodiments of FIG. 1.

FIG. 2B is an enlarged diagram of a portion of an output waveform such as that shown in FIG. 2A illustrating the modulation frequency on which such envelope is superimposed.

FIG. 2C is a diagram of the waveform of FIG. 2B after demodulation.

FIG. 3A is a block diagram illustrating one embodiment of a probe module to achieve multi-dimensional scanning.

FIG. 3B is a diagram of an alternative probe module for performing two or three dimensional scanning.

FIG. 3C is a diagram of an alternative probe module for achieving three dimensional scanning.

FIG. 3D is a diagram of an alternative probe module for performing circular scanning.

FIGS. 4A and 4B are diagrams of two additional probe module embodiments for performing multidimensional scanning.

FIG. 5 is a side sectional side diagrammatic view of one embodiment of an endoscopic probe module.

FIG. 6 is a side sectional diagrammatic view of a second embodiment of endoscopic probe module.

FIG. 7 is a side sectional diagrammatic view of a third embodiment of endoscopic probe module.

FIG. 8A is a diagram illustrating a first scan pattern for two dimensional scanning of a sample in accordance with the teachings of this invention.

FIG. 8B is a diagram illustrating a second scan pattern for two dimensional scanning of a sample in accordance with the teachings of this invention.

FIG. 8C is a diagram illustrating a third scan pattern for two dimensional scanning of a sample in accordance with the teachings of this invention.

FIG. 9 is a schematic block diagram of a parallel scanned embodiment.

FIG. 10 is a schematic block diagram of a balanced receiver embodiment.

FIG. 11 is a schematic block diagram of another fiber optic embodiment of the invention utilizing polarized light to detect birefringence.

FIGS. 12A-12C are diagrams obtained using an embodiment such as that shown in the figures to scan a human aorta which is normal, contains fatty plaque, and contains calcified plaque, respectively.

<u>Detailed Description</u>

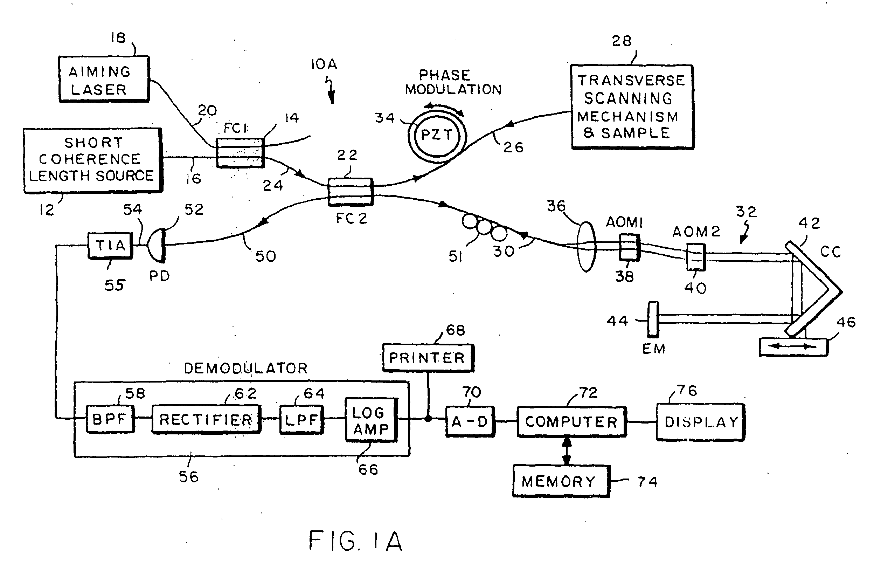

Referring first to FIG. 1A, an optical coherence domain reflectometer (OCDR) 10 is shown which incorporates the teachings of this invention. In particular, the output from a short coherence length (broad spectral bandwidth) optical source 12 is coupled as one input to an optical coupler 14. Such coupling may be through a suitable optical path, which for the preferred embodiment is a fiber optic path 16. Source 12 may, for example, be a light emitting diode, super luminescent diode or other white light source of suitable wavelength, or may be a short-pulse laser. Such sources preferably having a coherence length of less than 10 micrometers for preferred embodiments. As will be discussed later, it is desirable that the coherence length of source 12 be minimized to enhance the resolution of the system.

The other input to coupler 14 is from a laser 18 generating an optically visible output which is applied to the coupler through a fiber optic path 20. As is discussed in greater detail later, laser 18 does not contribute to the normal operation of the system and is utilized only to provide a source of visible light for proper alignment with a sample, when the light from diode 12 is in the infrared region and thus not visible.

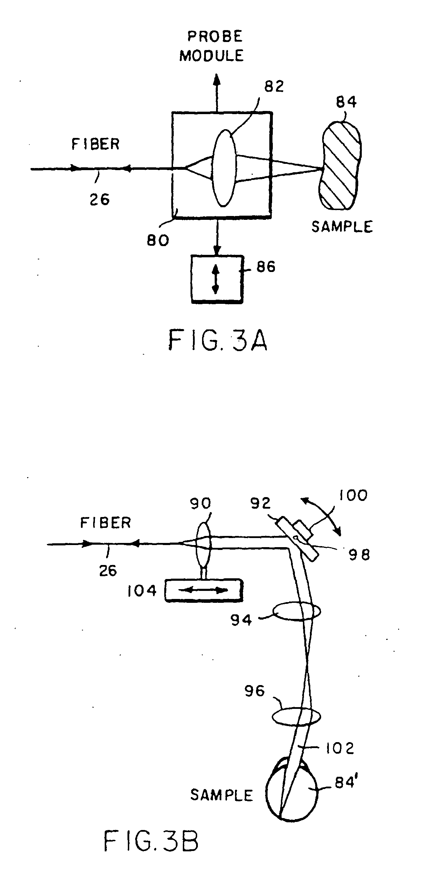

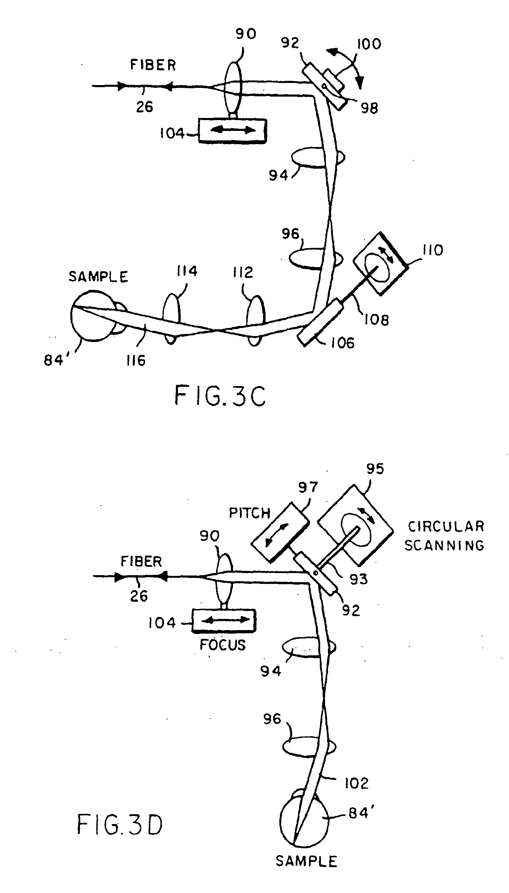

The output from coupler 14 is applied as an input to coupler 22 through fiber optic path 24. The light or optical energy received at coupler 22 is split between a first fiber optic path 26 leading to scanning/sample assembly 28 and a second fiber optic path 30 leading to a reference assembly 32. Assembly 28 may include a lens assembly formed of one or more lenses for focusing light received from optical path 26 on a sample to be scanned and various mechanisms for causing lateral, transverse or longitudinal motion of the light relative to the sample. In particular, while for the preferred embodiment longitudinal scanning is performed by movement at the reference assembly, it is also possible for the sample or probe to be moved longitudinally, or for longitudinal scanning to otherwise be performed at assembly 28. The assembly may also include a mechanism for controlling the longitudinal or depth position of the focus in conjunction with the longitudinal scan position. A probe module portion of assembly 28 may be designed for positioning adjacent to an outer surface of the sample, for example adjacent to a patient's eye for scanning and imaging or taking measurements on the patient's eye, or it may be adapted to be positioned inside the sample, being, for example, part of an angioscope or endoscope for scanning internal body or other channels. For purposes of FIG. 1A, the sample being scanned and/or imaged is included in the assembly 28. Various mechanisms which may function as the assembly 28 in accordance with various embodiments of the invention are shown in FIGS. 3-7.

For all embodiments, light transmitted by the probe to the sample is reflected by the sample back through the probe module to fiber 26. The optical fiber of path 26 may be wrapped around a piezoelectric crystal transducer or actuator 34 which vibrates (i.e. expands and contracts) in response to an applied electrical signal to cause slight expansion and contraction of the optical fiber and to thus modulate the optical signal passing through the fiber. As will be discussed later, this added modulation may facilitate detection.

Reference assembly 32 may include a collimating lens 36, first and second acousto-optic modulators 38 and 40 (AOM 1 and AOM 2), a corner-cube retroreflector 42 and an end mirror 44. For the preferred embodiment, corner cube 46 is mounted to a mechanism 46 which reciprocates the corner cube toward and away from both optical path 30 and end mirror 44 in a particular pattern to effect longitudinal scanning of the sample. As discussed in greater detail later, the corner-cube is preferably moved at a uniform, relatively high velocity (for example greater than 1 CM/SEC), causing Doppler shift modulation used to perform heterodyne detection. The length or extent of movement of cube 42 by mechanism 46 is at least slightly greater than half the desired scanned depth range in the sample. The scanning pattern for mechanism 46 preferably has a uniform velocity V, at least during the portions thereof during which scanning occurs, and may, for example, be a ramp pattern, or a sawtooth pattern. With a ramp pattern, measurements or imaging would be taken on the ramp while with a sawtooth pattern at velocity V on both sides, scanning can be done with the corner cube moving in either one or both directions. Further, a sine wave or other scan pattern can be utilized with suitable compensation in other elements of the circuit.

Alternatively, scanning in the longitudinal or depth dimension may be accomplished by reciprocating end mirror 44 with a suitable mechanism such as mechanism 46 rather than corner cube 42. However, if this is done, the effective stroke is reduced by 50% so that the end mirror 44 must be moved through a path which is slightly greater than the desired scan depth range rather than through a path equal to half such range. The greater travel stroke required for the mechanism 44 in this instance may adversely affect the scan rate achievable and may also limit the modulating Doppler shift frequency, requiring the use of additional modulating elements. If corner cube 46 is eliminated completely, the system becomes more susceptible to errors resulting from wobble of the end mirror as it is reciprocated.

It is also possible to eliminate the end mirror by arranging the corner cube for a single pass configuration. In this configuration, incoming light to the corner cube is aligned with the corner cube vertex. This also results in a 50% decrease in the effective stroke. In addition, as discussed above, mechanism 46 may be eliminated in the reference assembly 32, with longitudinal scanning being performed in assembly 28 by moving either the probe or sample in the longitudinal direction. This will be discussed later. If this is done, then corner cube 42 is not required and light from path 30 may inpinge directly on mirror 44.

Finally, while for preferred embodiments utilizing a Doppler shift frequency, mechanism 46 moves a corner cube or end mirror at a velocity which, as indicated above, is substantially constant in the scanning range, for some embodiments to be discussed, Doppler shift modulation in the longitudinal direction is not utilized and movement of the mirror is effected primarily to control the desired scan depth. For such embodiments and others, mechanism 46 may operate in step fashion to control the desired scan depth.

The total length of path 26 between coupler 22 and a selected depth point in a sample being scanned and the total length of path 30 between coupler 22 and end mirror 44, should be substantially equal for each depth point of the sample during a scan of selected depth range. In addition, to prevent group velocity dispersion which would decrease spatial resolution, the lengths of the optical fibers in paths 26 and 30 should also be substantially equal. Alternatively, the group velocity dispersion may be equalized by placing optical materials of known group velocity dispersion and thickness in the light paths to compensate for any inequality. For example, where the fiber in the reference path may need to be shorter than that in the sample probe, a length of high dispersion material may be included in the reference path. It is also important that the termination of the optical fibers utilized in this system be angle polished and/or anti-reflection coated to minimize reflections and maximize throughput.

Mechanism 46 may be any one of a variety of devices adapted for performing the translation function. For example, mechanism 46 could be a stepper motor, the motion of which is applied to corner-cube 42 or mirror 44 through an averaging mechanism for embodiments where uniform velocity is required. A DC servo-motor might also be utilized to obtain the desired motion. Various electromagnetic actuators, for example a speaker coil, may also be utilized for this function. With such electromagnetic actuators, detection of mirror position and servo control thereof may be required in order to achieve uniform motion where required. More specifically, in a uniform motion system, a signal indicative of desired mirror position at each point in the mirror travel path could be compared against a signal from a detector of actual mirror position and any resulting error signals utilized to control the actuator to maintain the mirror moving at the desired constant velocity. It is also possible to use a servo-control galvanometer driven linear translator for the mechanism 46.

One potential problem in the reference mechanism 32 is wobble of the mirror being translated which may adversely effect the accuracy of distance determinations. Such wobble is partially compensated for in the embodiment of FIG. 1A by corner-cube 42, such corner cubes generally having the property that, regardless of the angle at which a beam is incident thereon, the beam will always return in exactly the same direction at which the beam was incident. Other techniques known in the art may also be utilized to deal with the wobble problem.

Reflections received from assemblies 28 and 32 are applied through optical paths 26 and 30, respectively to optical coupler 22. These signals are combined in coupler 22, resulting in interference fringes for length-matched reflections, (i.e. reflections for which the difference in reflection path length is less than the source coherence length) and the resulting combined output is coupled onto fiber optic path 50.

To maximize interference between light returning from the reference and sample optical paths, their polarization should be substantially the same. To accomplish this polarization matching, a polarization controller may be placed in one of the optical paths 26 or 30. For purposes of illustration, a polarization controller 51 is shown in optical path 30 in FIG. 1A. Such a polarization controller compensates for changes in polarization in the fiber optic paths. Alternatively, polarization maintaining fibers and couplers may be utilized in the system to achieve the desired result. Further, in applications where polarization is randomly varying, a polarization diversity receiver can be utilized in the system to eliminate signal fading. Such polarization diversity receivers are known in the art.

The optical signal on fiber optic path 50 is applied to a photodetector 52 which converts the optical combined signal from path 50 to a corresponding current-varying electrical signal. The current-varying electrical signal on output line 54 from photodetector 52 is preferably converted to a voltage-varying signal by a transimpedance amplifier (TIA) 55 or other suitable means, the TIA output being applied as an input to a demodulator 56.

Various forms of demodulation may be utilized in practicing the teachings of this invention. In its simplest form, demodulator 56 may consist of a bandpass filter 58 centered around the modulation frequency of the combined output signal and an envelope detector. The filter assures that only the signal of interest is looked at and removes noise from the output. This enhances the signal-to-noise ratio of the system and thus system sensitivity. The filtered signal is then applied to the envelope detector.

The envelope detector in demodulator 56 may consist of a rectifier 62 and a subsequent low pass filter 64. The second filter removes any high frequency components from the base band signal. The demodulator may also include a logarithmic amplifier 66, either before or after the rectifier, for dynamic range compression. Where a logarithmic amplifier is not used, logarithmic compression may be performed elsewhere in the system, for example in the processing computer. Without logarithmic compression, strong reflections from boundaries would either be off scale or weaker reflections would not be visible.

The exemplary demodulator described above is one type of heterodyne demodulator. However, a variety of other demodulation techniques known in the art may also be utilized to perform the demodulator function.

The demodulated output from circuit 56 is the interferometric envelope signal of interest. A suitable printer 68 may be utilized to obtain a visual record of this analog signal which may be utilized by a doctor, engineer or other person for various purposes. For preferred embodiments, the analog output from demodulator 56 is applied, either in addition to or instead of to printer 68, through an analog-to-digital converter 72 to a suitable computer 74 which is programmed to perform desired analysis thereon. One or more memory devices 74 may be provided with computer 72. Computer 72 may, for example, control the display of the demodulated signal on a suitable display device 76, such as a cathode ray tube monitor, or may control a suitable printer to generate a desired record.

Where a printer or a computer display is utilized to reproduce the scanned image, characteristics such as density of the scanned image may be reproduced utilizing gray scale levels (i.e. dark for high density and light for low density) or a "false color" image may be generated with the color from blue to red across the color spectrum being indicative of the characteristic. In addition, computer 72 may detect various points of interest in the demodulated envelope signal and may perform measurements or make other useful determinations based on such detections. Computer 72 may be a suitably programmed standard processor or a special purpose processor may be provided for performing some or all of the required functions.

The OCDR shown in FIG. 1A may for some embodiment be utilized with corner cube 42 being scanned by mechanism 46 at an Intermediate but uniform velocity. For purposes of this discussion, an intermediate scanning velocity is considered one at which the Doppler frequency shift caused by cube or mirror movement is not negligible, but is low enough to fall within the predominant low frequency noise for the system. The noise spectrum includes noises arising from fluctuations in source 12, mechanical components and electrical circuits, and are larger at lower frequencies, typically below 10 kHz. High scan velocity is considered to be a velocity where the Doppler frequency shift is higher than the predominant low frequency noise. The Doppler shift frequency $f_D$ results from the translation of the cube 42 and, with a corner-cube, is given by the equation: $f_D \sim 4V/\lambda$ where V is the velocity at which the cube is being moved at the given time and $\lambda$ is the optical wavelength of the source. Where a corner-cube is not used, $f_D \sim 2V/\lambda$. Thus, in addition to compensating for wobble as the mirror is translated, the corner-cube also doubles the Doppler shift frequency, and effective scanning stroke, for a given velocity V of the mechanism 46.

Where this Doppler shift is less than the required bandwidth to overcome noise, including where stepped longitudinal or no longitudinal scanning is being performed so that the Doppler shift frequency is substantially zero, additional modulation is needed to shift the modulation frequency above the predominant noise spectrum. In FIG. 1A, this may be achieved by introducing sinusoidal phase modulation by use of piezoelectric transducer 34. While in FIG. 1A the additional modulation is introduced by use of the oscillator or transducer in sample path 26, such modulation could also be provided in the reference arm or path 30. Equivalent piezoelectric modulation of end mirror 44 could also be utilized. Further, in addition to piezoelectric transducer 34, the small movement required for this supplemental modulation may be achieved using electromagnetic, electrostatic, or other elements known in the art for providing small generally sine wave movements.

Alternatively, as shown in FIG. 1A, this supplemental modulation can be achieved by passing light in the reference arm and/or sample arm through acousto-optic modulators (AOM's). Such modulators produce a frequency shift of the light beam and thus produce an effect substantially equivalent to Doppler shifting the beam. Such acousto-optic modulators can, in some instances, be substituted for the movement of the mirror or corner cube. The AOM'S, which can be bulk optical devices as shown in FIG. 1A or, may be smaller in-line optical fiber AOM's, effectively raise the carrier frequency to allow for high speed scanning. While one AOM may be adequate for this purpose, two AOM's can be used as shown in FIG. 1A. The reason for two AOM's is that since AOM's are normally driven at a much higher frequency then is required for this application, the detection frequency can be lowered to a desired frequency by driving two AOM's at different frequencies, the detector frequency being the difference frequency.

Supplemental modulation from element 34, or from other suitable means which modulate the optical path length, is at a frequency $f_M$ and the oscillation amplitude of this modulator is adjusted so that the peak-to-peak oscillating movement or optical delay change is approximately one-half of the wavelength $\lambda$ of source 12. The combined effect of the supplemental modulation and the Doppler shift frequency causes the output envelope to be at modulating frequencies of $f_D$, $f_M + f_D$, $f_M - f_D$ and at higher harmonics of $f_M \pm f_D$. $f_M$ is normally chosen to be high enough to overcome noise spectrum and aliasing problems.

Demodulation of the output from photodetector 54 is normally at $f_M + f_D$ and/or $f_M - f_D$. For purposes of illustration, it will be assumed that demodulation is at $f_M + f_D$. The center frequency for bandpass filter 58 is thus set for the frequency ($f_M + f_D$). The bandwidth for filter 58 should be approximately two to three times the full-width-half-maximum (FWHM) bandwidth of the received signal to avoid signal broadening and distortion. The bandwidth of low pass filter 64 would typically be roughly identical to that of bandpass filter 58. While if the velocity at which cube 42 is being moved has a high enough speed so that the resulting Doppler shift frequency is higher than the predominant noise spectrum and wide enough so that transverse scanning does not cause signal aliasing, then supplemental modulation by devices such as modulators 34, 38, 40 is not required, this may not be possible with two-dimensional or three-dimensional scanning because of the broad bandwidth involved.

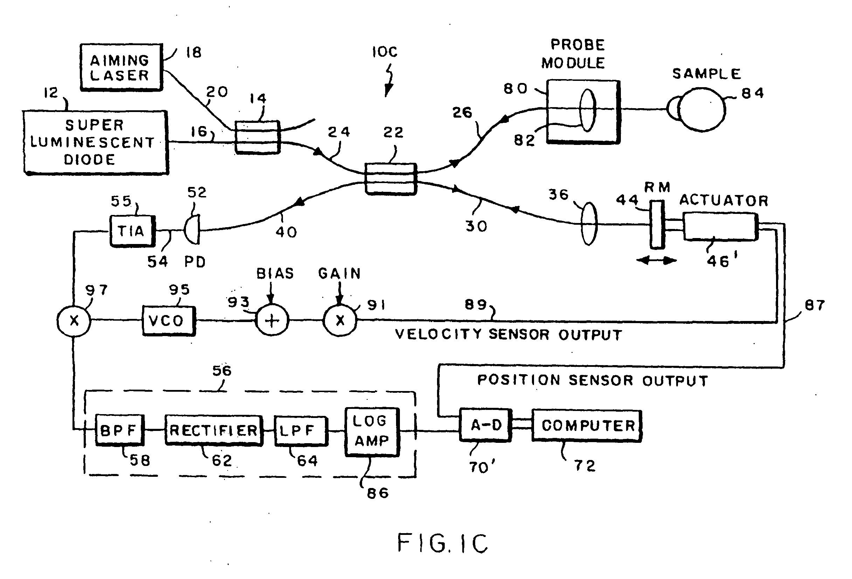

For the embodiments discussed to this point, the scanning of cube 42 has been at constant velocity, at least through the scan interval. However, for very high speed scanning at high scan repetition rates that cannot be realized with servo-controlled constant velocity mechanical drives, resonantly (sinusoidal) driven mechanical actuators can be used to drive cube 42 or mirror 44. These actuators can be galvanometrically or electrodynamically driven at the resonant frequencies of the mechanical actuator system and are commercially available. Adjustments to the system required to accommodate a sinusoidal drive are discussed later in conjunction with FIG. 1C. Alternatively, where higher speed scanning is required, electro-optic techniques could be used in lieu of mechanical techniques to effect scanning. For example, an acousto-optic modulator or other electro-optic modulator could be utilized to vary the light path. However, such devices are currently expensive and have limited range; such devices would therefore not be preferred for most applications.

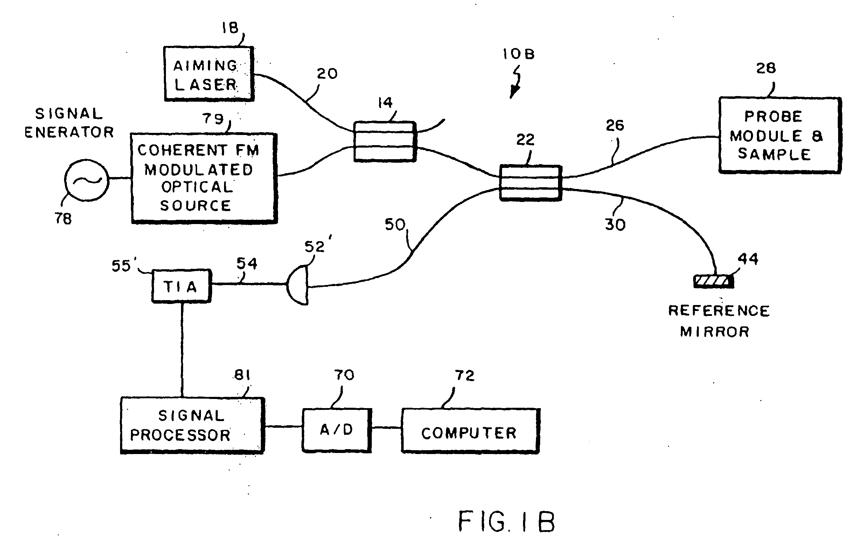

FIG. 1B illustrates an alternate embodiment of the invention wherein longitudinal ranging information is obtained by optical frequency domain reflectometry rather than by optical coherence domain reflectometry. In this figure, and in the remaining figures, the same reference numerals are utilized to identify common elements. A prime number may be utilized to identify a common element with a prior figure where the element has been slightly modified.

FIG. 1B shows an optical frequency domain reflectometer utilizing a spectrally coherent optical source 79 which is frequency modulatable in one of a number of ways known in the art. Source 79 is frequency modulated in the form of a linear FM chirp by signal generator 78. The output from source 79 passes through the same optical paths described in conjunction with FIG. 1A to a sample assembly 28 and to a reference mirror 44. Since changes in optical path length are not being utilized for this embodiment of the invention to perform longitudinal scanning, the remainder of the reference assembly shown in FIG. 1A is not required nor are modulators 34, 38 and 40. A lens such as lens 36 may or may not be required.

Reflected radiation from the sample in assembly 28 and from reference mirror 44 are combined in fiber optic coupler 22 and directed through optical path 50 to a wide bandwidth photodetector 52' where they optically interfere. Wide bandwidth photodetector 52' and transimpedence amplifier 55' are used to amplify the detected signal. The detected optical interference generates an RF frequency that is proportional to the differential path length between the sample reflection and the reflection from reference mirror 44. A variety of methods known in the art exist to convert this frequency information into spatial information in an electrical processor 81. These include using a waveform recorder with inverse Fourier transform techniques. Requirements on and techniques to achieve linearity, spectral coherence, modulation bandwidth and frequency deviation are all known in the art and such techniques can be employed in the embodiment of FIG. 1B. The output from processor 81 is digitized using A/D converter 70 and processed by computer 72 in the manner discussed in conjunction with FIG. 1A. Printers and displays may be provided for this embodiment of the invention as for the embodiment shown in FIG. 1A. With suitable modifications, the teachings of the invention may also be practiced employing a linearly chirped intensity modulated source.

Where, as discussed above, actuator 46 has a sinusoidal or other non-linear velocity profile, the Doppler shift frequency $f_D$ is no longer constant, and the demodulator 56 must be adapted for this carrier frequency variation. There are least two methods for accomplishing this objective. In both cases, as illustrated for system 10C in FIG. 1C, an output line 87 is provided from a position sensor in the actuator 46. For simplicity, the reference assembly in this figure is shown as an end-mirror 44 moved longitudinally by an actuator 46'. The voltage on line 87 will normally vary as a function of actuator position and thus of position for mirror 44; however, the position sensor output may also be current varying. If the sensor provides a digital output, then line 87 may be connected to computer 72 without going through A/D converter 70'.

The signal on line 87 is required when actuator 46' has a non-linear velocity profile so that intensity and other inputs received at computer 72 may be correlated with scan position in the sample. This correlation is not required with a linear scan where position can be determined from the time an input is received.

In the simpler technique, the acceptance band for bandpass filter 58 and low pass filter 64 are increased to accommodate the variations in the Doppler shift frequency $f_D$ over a large portion of the sinusoidal motion of mirror 44. These variations occur because $f_D$ varies directly with variations in V. This increased demodulator acceptance bandwidth will lead to increased acceptance of noise and thus results in lower detection sensitivity. However, this technique is simple and can be used in cases where the requirement for detection sensitivity is not critical. Further, this increase in acceptance bandwidth may be relatively small when the signal bandwidth $\Delta f_{FWHM}$ is already large relative to $f_D$, this occurring when the coherent length is very small.

FIG. 1C illustrates the second technique wherein the demodulation frequency is dynamically tuned to the instantaneous Doppler shift frequency using a superheterodyne system. A sensor at actuator or drive mechanism 46' provides a velocity dependent voltage on line 89 which is modified by a gain circuit 91 and a bias circuit 93 before being applied to a voltage controlled oscillator 95. The output from oscillator 95 is multiplied in a circuit 97 with the output from detector 52 via amplifier 55. The gain and bias of the signal applied to VCO 95 are adjusted so that the modulating frequency at the output from multiplier 97 is substantially constant at a desired center frequency which is selected as the center frequency for bandpass filter 58. As with the embodiment of FIG. 1A, the bandwidth of filter 58 is set at two to three times the peak signal bandwidth and, except for the need for the position sensor output on line 87, the remainder of the detection and processing would be substantially identical to that previously described in conjunction with FIG. 1A.

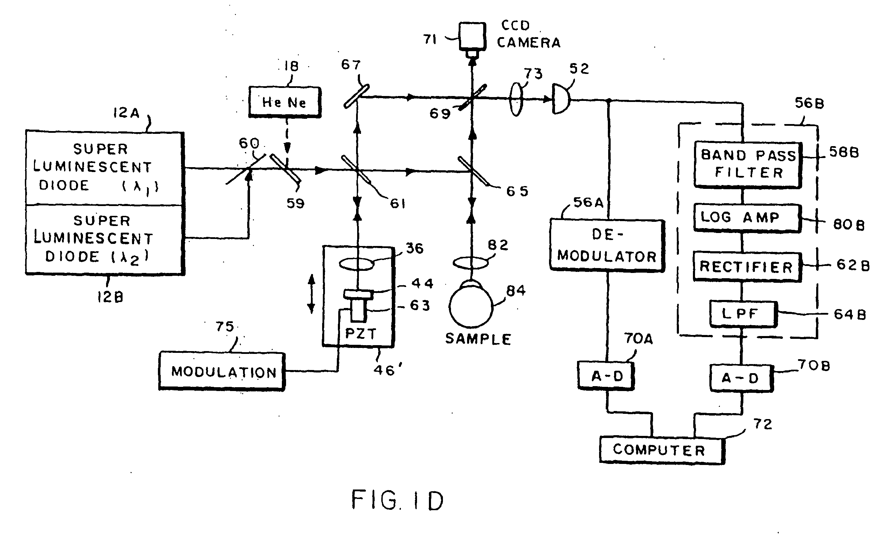

FIG. 1D shows a system 10D which is similar to that of FIG. 1A, except that bulk optics are utilized rather than fiber optics and ability to observe spatial properties is enhanced by providing two light sources 12A and 12B which are at different wavelengths. While the multiple wavelength option is being shown for purposes of illustration in conjunction with a bulk optics embodiment, it is to be understood that multiple wavelengths could also be, and may preferably be, used with the fiber optic embodiments. Sources 12A and 12B could be the same type of light sources designed to operate at different wavelengths or could be different types of light sources. The outputs from sources 12A and 12B are merged in a coupler 60, the optical output from which Is applied to a coupler 59. The other input to coupler 59 is the output from a laser 18, for example, a helium neon laser, which again is used only for alignment purposes. Couplers 60 and 59 could, for example, be dichoric beam splitters, polarization beam splitters or normal beam splitters.

The output from coupler 59 is applied to beam splitters 61 and 65. Beam splitter 61 applies a portion of its input through lens 36 to mirror 44 and also passes optical radiation to beam splitter 65 which applies this radiation through lens 82 to sample 84. Reflections from mirror 44 are applied through lens 36, beam splitter 61 and mirror 67 to interferometric coupler 69. Mirror 44 and lens 36 may be part of a translation stage which is moved by a mechanism such as the mechanism 46' discussed in conjunction with FIG. 1C. As was dicussed above, if such translation is being performed at a speed such that $f_D$ is below the predominant noise spectrum, it may also be necessary to, for example, mount mirror 44 to a piezoelectric crystal 63 which is vibrated under control of a modulator 75. Other methods for accomplishing this are discussed earlier. Reflections from sample 84 are applied through lens 82 and beam splitter 65 to the interferometric coupler 69.

The output from coupler 69 may be applied to a CCD camera 71 used for alignment purposes and is also applied through a lens 73 to a photodetector 52. The output from the detector is applied through two separate paths. Each path contains a demodulator 56A, 56B containing a bandpass filter 58 having a center frequency which corresponds to the Doppler shift frequency $f_D$ for the given source 12. Since $f_D$ varies inversely as a function of the source wavelength, each demodulator only demodulates signals corresponding to the appropriate source wavelength, permitting outputs resulting from the two source wavelengths to be separated. After being applied through corresponding A-D converters 70, the two outputs are applied to computer 72 where they may be appropriately processed.

Alternatively, a detector 52 may be provided corresponding to each source wavelength, where each photodetector is preceded by an optical wavelength filter that only transmits the appropriate wavelength with an appropriate pass band. A beam splitter would be provided ahead of the optical wavelength filters, with a demodulator at the detector output.

While in FIG. 1D, and in the discussion above, only two separate signals $\lambda$ have been shown, this is not a limitation on the invention, and a greater number of light sources and detectors (and/or demodulator circuits) may be provided for appropriate applications.

For purposes of describing the operation of the systems, for example 10A or 10D, it will be assumed that the sample 84 is the eye of a human or animal patient. When such measurements are to be made, there are three alignments which are critical. First, the beam must be aligned with the sample so that it enters the sample at a desired angle. This angle is normally an angle perpendicular to the angle of the eye layers. Second, the beam must be laterally positioned on the sample area of interest. This is a control of the lateral position of the beam. Finally, the beam must be focussed at the level of interest in the eye. A number of techniques may be utilized for performing each of these alignment functions.

In particular, a number of different techniques may be utilized to obtain a desired incidence angle. Since reflections will generally be substantially maximized when the beam is normal to the layer or surfaces being reflected off of, one simple way to achieve alignment is to adjust the position or angle of the probe 80, of beam splitter 65, or lens 82 and/or of the sample (i.e., the patient's eye) and, with the reference arm blocked, detect reflections from the sample. The alignment at which the power of the detected reflections is maximum would thus be the desired alignment angle. it would normally be possible to locate the desired angle relatively quickly using this technique.

A second technique for achieving angular alignment is similar to the first except that the reference arm is not blocked and, with normal readings being taken from the system, alignment is manually adjusted until an alignment which maximizes the output is obtained.

A third method is to look at the direction in which the beam is reflected in order to detect beam alignment. Since it is hard to do this directly, particularly when a fiber is utilized, such determination is generally made by providing a beam splitter which directs a portion of the beam reflected from the sample to a device such as CCD camera 71 (FIG. 1D) which can measure beam position. This device is initially calibrated with the system so that the spot on which the beam impinges on the camera when the beam is properly aligned with a sample is determined. Then, in operation, the sample and probe can be adjusted until an angle of alignment is achieved where the beam impinges on the CCD camera at the previously determined point.

Lateral position alignment is at this time best performed manually. To perform this operation, laser 18 is turned on. Source 12 may either be on or off for this operation. Laser 18 provides a narrow beam visual indication of the lateral position on the eye where the beam is striking and the position of either the probe beam or the patient may then be manually adjusted until the beam is striking the desired position. If light from source 12 is in a visible band, laser 18 may not be required and light from source 12 may be used for alignment.

The focussing cone angle to be utilized for performing readings is determined by balancing the desirability of having as large a numerical aperture (cone angle) as possible against being able to achieve a desired longitudinal range or depth of field in which back scattered or reflected light is efficiently coupled back to the fiber (or to the other optical path 26 where a fiber is not employed). A large numerical aperture makes angular alignment for normal incidence on the sample surface less critical and for measurement of back scattering where the returned radiation is spread over wide solid angles, a wider cone angle increases the coupling into the fiber. However, the large cone angle reduces the longitudinal range. Thus, the numerical aperture or f number should be selected to correspond to a depth of field that is equal to the longitudinal extent of the area in the eye or other sample on which measurements are to be taken. For purposes of this discussion, depth of field is defined as the longitudinal distance from the focal plane at which the back coupling efficiency into the fiber is reduced by one-half.

As for the other alignments, the sample and/or probe are moved relative to each other until the system is focussed to a desired point within the sample, i.e., within the eye. Since even with the laser it may be difficult to visually determine the focal point, a preferable way to perform focussing may be to operate the system with, for example, an output being obtained on display 76. As will be discussed later, certain high amplitude points in such output are indicative of a particular layer or transition in the eye and focus can be adjusted until this transition occurs at a desired point in the scan.

Once alignment has been achieved, the system may be utilized to take desired measurements. To perform such measurements, aiming laser 18 is turned off and source 12 is turned on. Mechanism 43 or 43' is also turned on, if not already on, to cause desired movement of the cube or mirror. If mechanism 43,43' is not moving at sufficiently high velocity, it may also be necessary to turn on piezoelectric modulator 34 or 63.

As previously indicated, source 12 should have a low coherence length which implies being spectrally wide. Thus, for light sources of the type previously mentioned having a coherence length of approximately 10 micrometers, spatial separation, and thus resolution, to 10 micrometers can be obtained. This is a far higher resolution than is available with other currently available devices.

Path lengths 26 and 30 are initially equal with the beam focussed at a desired initial scan depth in sample 28. As mirror 44 (or cube 42) is moved away from lens 36, the point in the sample at which the path lengths are equal is scanned to successively greater depths within the sample. At each point in the scan, reflections occur and light scattering occurs which are a function of the refractive index variation for the material through which the light is passing and of such index boundaries. Interference fringes occur for depth points in the sample where the difference between the path length to the point in the sample ($L_s$) and the path length to the current mirror location ($L_m$) differ by less than the coherence length (CL) of the light source (i.e. $L_s - L_m < CL$). Therefore, the coherence length of the light source determines available system resolution. This is the reason for keeping coherence length as low as possible.

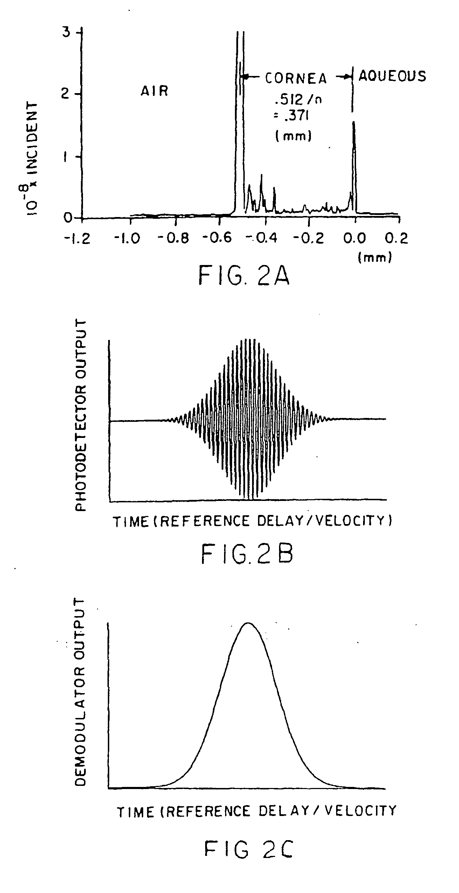

The interferometric output from coupler 22 or 69 is thus indicative of reflections or scattering obtained at a particular depth within the sample. The successive interferometric outputs obtained during a scan form an envelope signal such as that shown in FIG. 2A which normally has peaks at optical junctions within the samples where reflections are normally maximum and may have some lesser peaks in a predetermined pattern, depending on the scattering characteristics of the medium at the scan depth.

When the mirror is being scanned at a velocity V, a Doppler shift frequency having a frequency $f_D \sim 2V/\lambda$, ($\sim 4V/2$ for FIG. 1A where corner cube is moved) where V is the velocity at which the mirror is moved and $\lambda$ is the wavelength of source 12, is superimposed on the envelope signal as shown for a small portion of an intensity output in FIG. 2B. FIG. 2C shows this same output portion after demodulation.

From the equation indicated above, it is seen that the Doppler shift frequency is dependent on the wavelength of source 12. Thus, for the embodiment shown in FIG. 1D, where two separate optical energy sources 12A and 12B are provided, the interferometric output from coupler 69 will contain two separate envelopes which are a function of the differences in absorption and reflection at the different wavelengths, and each interference output will be modulated at a different Doppler shift frequency. Thus, as previously indicated, the bandpass filter 58 in each demodulator 56 may be selected to have a center frequency and bandwidth for a different one of the Doppler shift frequencies, or optical filtering with multiple detectors may be utilized, to permit detection and separation of these two signals.

The ability to perform the interferometric detection at two or more different wavelengths offers unique advantages. These advantages arise from the fact that the absorption, reflection and other optical characteristics of various sample materials vary with wavelength. Thus, taking measurements at two or more wavelengths permits the spectral characterization of optical properties of the sample such as the wavelength dependent absorption and scattering thereof. In particular, the log rate of attenuation of back scatter is different for different materials and, for a given material, may vary with wavelength. By observing the back scatter pattern at different wavelengths from a substance, and possibly by observing the average rate of back scatter or reflection attenuation from layers of the sample, information concerning the material of the layer or various properties of such material may be obtained. The measurement of various spectral properties may be of interest in themselves and may also be used to distinguish between two sample layers, for example, two tissue layers that it is normally difficult to distinguish with single wavelength measurements because of their similar optical properties. In particular, by taking ratios at each of the wavelengths, spurious effects such as misalignment are compensated for permitting boundaries to be more easily and accurately identified. Basically, such boundaries are identified by looking at ratios rather than absolute values.

FIG. 3A illustrates one relatively simple embodiment for the assembly 28 of FIGS. 1A-1B. For this embodiment, fiber 26 terminates in a probe module 80. The probe module includes one or more imaging lenses, a single lens 82 being shown in the figure, positioned between the output of fiber 26 and the sample 84 being scanned. A suitable linear translation stage or other mechanism 86 is connected to move probe module 80 either transversely or laterally relative to the sample 84 to provide two-dimensional scanning. A similar mechanism (not shown) may be provided to move the probe in the other of the transverse or lateral direction to provide three-dimensional scanning of sample 84. (Transverse and lateral scanning are sometimes collectively referred to hereinafter as transverse scanning.) Mechanism 86 may be a stepper motor or other suitable positioning mechanism and is preferably controlled either by computer 72 (FIG. 1) or by a positioning computer which also provides positioning information to computer 72 so that the position of the scan on sample 84 is known by the computer. Further, as previously discussed, either probe module 80 or sample 84 may be moved in the longitudinal direction by a suitable translating mechanism to effect longitudinal position for scanning. This would be done in lieu of or in conjunction with moving the corner cube or end mirror.

FIG. 3B shows another embodiment of the invention wherein the probe module includes a first collimating lens 90, a steering mirror 92 which may be rotated by a galvanometer or other suitable mechanism 100 about one or two axes in a plane sometimes referred to as the pupil plane, and two additional focusing lenses 94 and 96. The sample is illustrated as an eye 84'. For FIG. 3B, focus is at or near the rear of eye 84' and the beam is pivoted about the nodal point of the eye at roughly the position of the ocular lens to scan different points along the rear surface of the eye as mirror 92 is rotated about pivot 98 and/or a pivot perpendicular thereto by mechanism 100. Again, the position of mirror 92, would be communicated to computer 72 in a suitable manner.

Further, as previously discussed, as corner cube 42 is moved by mechanism 46, the longitudinal or depth point in eye 84' at which detection occurs is varied. However, as is seen in FIG. 3B, the depth of the focal point for the light beam 102 in the eye remains constant. Thus, for much of a given depth scan, the beam 102 is out of focus with the point at which readings are being taken. To overcome this problem, a scanning mechanism 104 is provided which is synchronized with scanning mechanism 46 and which moves focusing lens 90 in a direction parallel to the direction of the light passing therethrough. This causes a change in the focal depth for beam 102 in sample 84'. With drives 46 and 104 synchronized, the focal point of beam 102 in eye 84' can be made substantially equal to the point being scanned in the eye at each point in time, providing optimum resolution for measuring and imaging. Other techniques known in the art for altering focal point in the longitudinal direction could also be utilized to synchronize the focal and detection points.

In FIG. 3B, scanning is provided in one or two transverse dimensions in a single pupil plane. FIG. 3C shows an embodiment wherein two single-axes scanning mirrors are used, an additional scanning mirror 106 being provided in a second pupil plane, which mirror is scanned about a shaft 108 by a galvanometer or other suitable mechanism 110 in a direction perpendicular to the direction of rotation of mirror 92. Light reflected off of mirror 106 is passed through lenses 112 and 114 to pass through the aperture of eye 84' to a selected focal point in the eye, which point may be varied in three dimensions. The galvanometer-driven mirrors 92 and 106 in FIGS. 3B and 3C could be replaced by rotating polygonal mirrors or other angular beam steering devices. As with prior embodiments, information concerning positions is communicated to computer 72 to permit proper imaging and processing.

FIG. 3D shows still another embodiment of the assembly 28 wherein the mirror 92 in the pupil plane is rotated about a shaft 93 by a suitable rotary motion mechanism 95 and also has its pitch altered by a pitch altering mechanism 97. This results in a circular scan of eye 84', the size (i.e. diameter) of the circle being scanned depending on the pitch angle of mirror 92. The mechanism of FIG. 3D may for example be utilized to scan around the optic nerve head of the patient's eye and this scan may be processed to provide a two-dimensional scan. Although mechanical steering mechanisms have been discussed above, electro-optic steering mechanisms known in the art could also be utilized.

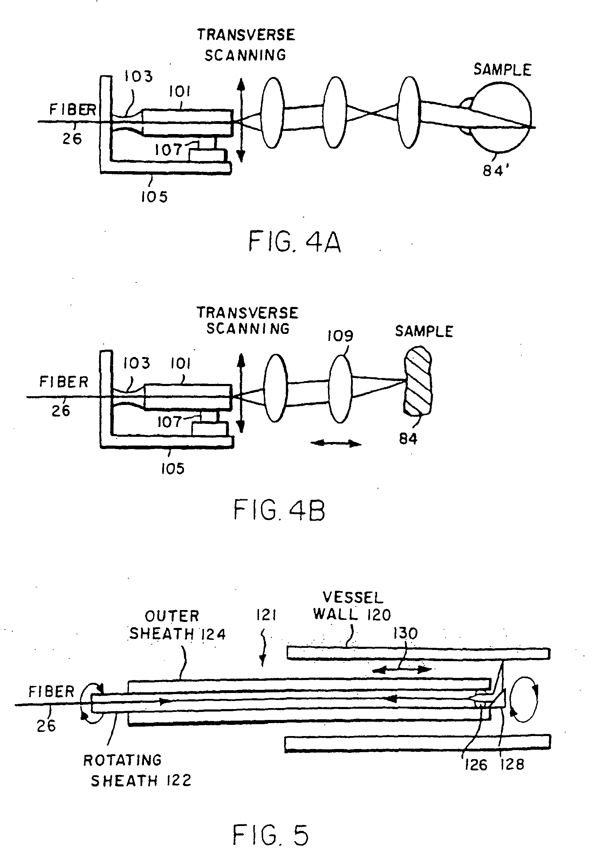

FIGS. 4A and 4B show still another embodiment for assembly 28 wherein fiber 26 is embedded in a sheath 101 which is attached to a stationary housing 105 through a pivot joint 103. Sheath 101 rests on a mechanism 107 fixed to housing 105 which mechanism may for example be a piezo-electric crystal, stepper motor, electro-magnetic actuator, electro-static actuator, or the like. As mechanism 107 moves sheath 101, the tip of fiber 26 is moved transversely. This movement is converted by lenses to either an angular scan about a fixed entry point in eye 84' (FIG. 4A) and hence a transverse scan at the focal plane of the eye, or a transverse scan along a sample such as sample 84 (FIG. 4B). Lens 109 is shown as being movable longitudinally in FIG. 4B to either control longitudinal scanning or to synchronize the focusing in sample 84 with a longitudinal scan being performed in one of the manners mentioned previously in conjunction with FIGS. 1A-1B. If desired, pivot 103 may be eliminated so that sheath 101 moves straight up and down as a result of the operation of mechanism 107 rather than moving in an angular direction.

FIG. 5 illustrates an alternative embodiment wherein the probe module is part of an angioscope or endoscope which may be utilized for imaging tubular structures 120 such as blood vessels, the esophagus, or the like. The distal end of fiber 26 is embedded in an inner sheath 122 which is rotatably mounted within an outer sheath 124. Inner sheath 122 has a lens 126 formed therein at the distal end of fiber 26 and terminates in an angled mirrored surface 128, which surface extends beyond the end of outer sheath 124. The probe module 121 may be moved laterally along vessel wall 120 (i.e. in the direction of arrow 130) either manually or by a suitable drive mechanism to scan the vessel wall in one dimension, and inner sheath 122, including mirror 128 which forms part thereof may be rotated relative to outer sheath 124 to scan the vessel wall in a second dimension. The movement of corner cube 42 under control of mechanism 46 causes scanning in the depth dimension of the vessel wall to provide three-dimensional scanning, or such scanning in the depth dimension may be achieved by one of the techniques described above. Since for the embodiment shown in FIG. 5, probe module 121 may move a substantial distance in the direction 130 along the vessel wall, in order to maintain the desired equal length for paths 26 and 30, fiber 26 may initially be provided with a certain amount of slack or may be curled or coiled to permit movement in this direction.

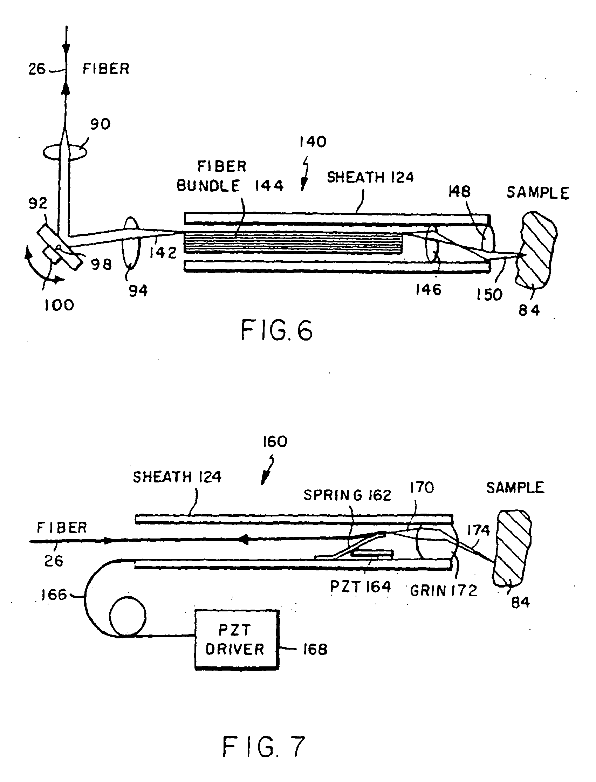

The endoscope probe 140 shown in FIG. 6 has a lens 90 at the distal end of fiber 26, a galvanometer-controlled mirror 92 and a focusing lens 94 which function in the same way and perform substantially the same functions as the corresponding elements in FIG. 3B. Output beam 142 from lens 94 is applied to a selected one or more of the single-mode optical fibers in fiber optic bundle 144. The fiber of bundle 144 to which beam 142 is applied depends on the scan position of mirror 92. At the distal end of fiber bundle 144, the output from the bundle 144 is passed through lenses 146 and 148 to sample 84. The transverse position of the scanning beam 150 on sample 84 varies with the fiber in bundle 144 to which beam 142 is applied and thus with the position of mirror 92. Beam 150 may thus be linearly scanned across sample 84 by the rotation of mirror 92. If mirror 92 is scanned in two dimensions, or if the output from lens 112 of the three-dimensional scanning mechanism of FIG. 3C is used instead of the output from lens 94, and fiber optic bundle 144 has fibers in two dimensions rather than in a single dimension, beam 150 may be scanned in a two-dimensional pattern across the surface of sample 84, permitting three-dimensional scanning to be performed.

FIG. 7 shows still another endoscopic probe module 160 which may be constructed utilizing the teachings of this invention. For this embodiment, the distal end of fiber 26 is connected by a spring 162 to the inner wall of sheath 124. Spring 162 rests on and is vibrated by a piezoelectric transducer 164, or other electromagnetic, electrostatic or other actuator known in the art, which is connected by electric cable 166 running along the wall of sheath 124 to a driver 168. Transverse motion of fiber 26 causes a corresponding transverse movement of light beam 170 which is applied to a graded refractive index lens (GRIN lens) or other suitable lens 172. The output light beam 174 from lens 172 provides a transverse scan of sample 84.

While three different configurations of angioscopic/endoscopic probes are shown in FIGS. 5-7, it is apparent that the teachings of this invention may be utilized to provide other angioscopic/endoscopic probe modules with either internal or external optics, with movement of either the fiber itself or of an external lens or mirror, and with varying scan patterns depending on application.

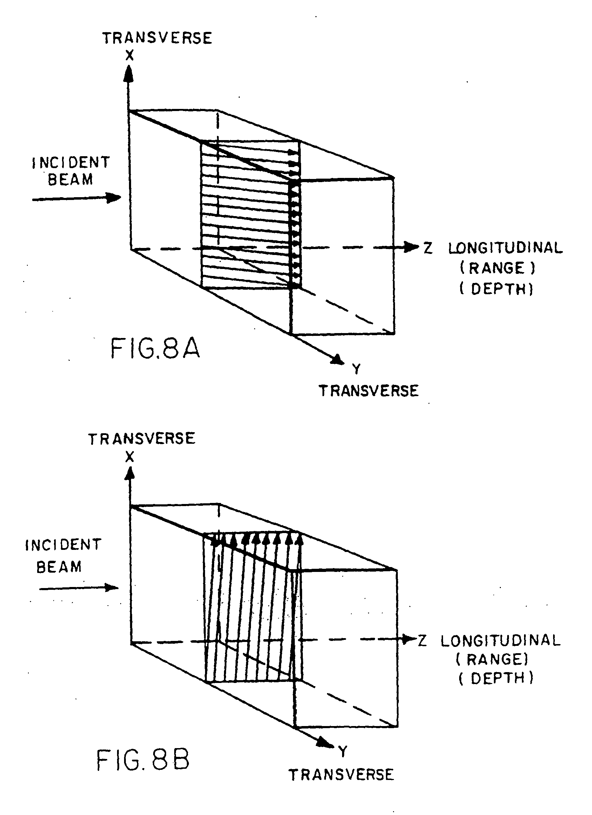

As was discussed earlier, a typical scan pattern for the various embodiments of the invention is for the probe assembly to be positioned at a selected transverse position with respect to the sample and mechanism 46 or other longitudinal scanning mechanism discussed in conjunction with FIGS. 1A-1B to be operated to complete the longitudinal or depth scan at the given transverse position. The transverse position is then altered in a manner, for example, described in conjunction with FIGS. 3-7 and a depth scan is completed at the new transverse position. This process is repeated until scanning has been performed at all desired transverse positions. This is the scan pattern illustrated in FIG. 8A.

However, the scan pattern shown in FIG. 8A requires high velocity longitudinal scanning. As discussed above, for some embodiments this longitudinal scanning is preferably at a constant velocity in order to produce a uniform Doppler shift which can be demodulated in circuit 56 (FIG. 1A). However, very high speed constant velocity scanning is difficult to achieve. Therefore, since there is less of a requirement on constant velocity for transverse scanning, and since resonantly driven galvanometers or fiber deflectors can be used to produce very high rates of transverse scanning, a scan pattern such as that shown in FIG. 8B may be preferable, particularly when a large number of transverse points are being utilized for the image. In FIG. 8B, a complete transverse scan is performed for each longitudinal position. In other words, referring, for example, to FIG. 3A, mechanism 86 would perform a complete cycle for each position of mechanism 46 (FIG. 1A). With this scan pattern, mechanism 46 could be stepped rather than continuously slewed.

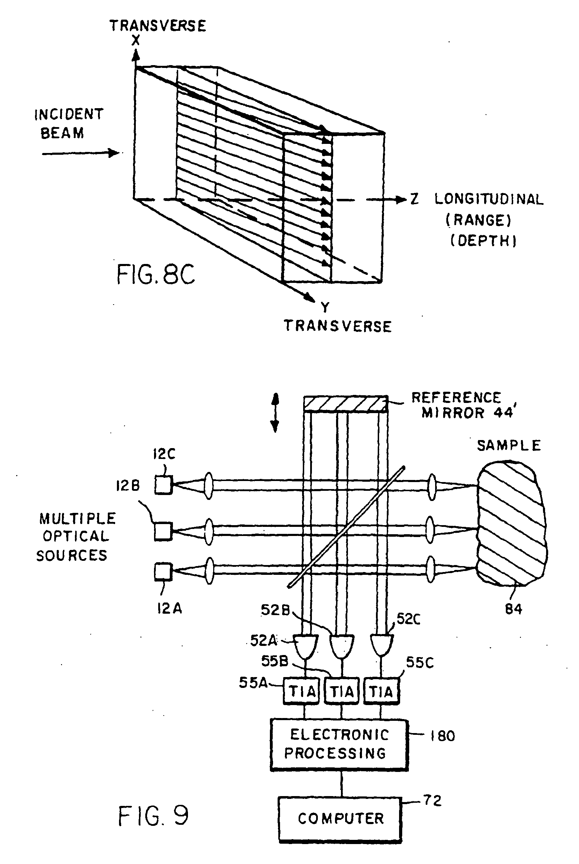

FIG. 8C shows still another scan pattern which may be utilized in practicing the teachings of this invention. In this scan pattern, the longitudinal position in the sample is controlled using one of the techniques for longitudinal positioning previously discussed, for example by stepping the position of end mirror 44 to a selected position, and scanning is then performed at this depth or longitudinal position in the sample in one or two transverse dimensions. Once such a scan has been completed, the scan may either be repeated at the same depth or the longitudinal position control may be stepped to cause the subsequent scan to be performed at a different depth. It should be noted that this three-dimensional scan is similar to that of FIG. 8B except that scanning at each depth level is performed in two rather than one dimension and such two-dimensional scans may be performed at only one or more selected depths rather than at all selected depths.

In the foregoing description, the scan pattern in the transverse dimensions need not be performed using straight lines. Curved or circular scan patterns may be used in instances where it is desired to obtain depth and cross-sectional image information along specific surfaces which are not curved. The scanning embodiments of FIGS. 3D and 5 illustrate this point.

One potential difficulty with the embodiments of the invention discussed up to this point is that a complete two or three-dimensional scan of a sample may take a substantial period of time. While this may be acceptable for samples which do not change with time, such as certain mechanical or semiconductor samples, it may not be acceptable for biological samples which may change rapidly with time. FIG. 9 illustrates an alternative embodiment of the invention wherein this problem is overcome by scanning the sample in parallel using multiple optical sources 12A-12C and multiple detectors 52A-52C, but a single movable reference mirror 44'. Separate optical sources may be provided for each source 12A-12C or optical radiation from one or more sources may be divided to provide the desired number of optical sources. Similarly, multiple references may be provided. The outputs from the multiple detectors 52A-52C are processed by a special processing circuit 180 before being applied to computer 72. Where a small number of parallel scans are being performed, it may still be necessary to also scan such sources laterally. For example, each of the beams applied to sample 84 in FIG. 9 could also be scanned in the direction in and out of the figure to provide three-dimensional scanning. Alternatively, parallel scanning could be performed in three dimensions. Assuming the capacity of electronic processing circuitry 180 is adequate, a large enough number of parallel scans in two or three dimensions could be provided so that additional lateral or transverse scanning of the beams is not required. Parallel scanning could also be performed utilizing the scan technique of FIG. 1B.

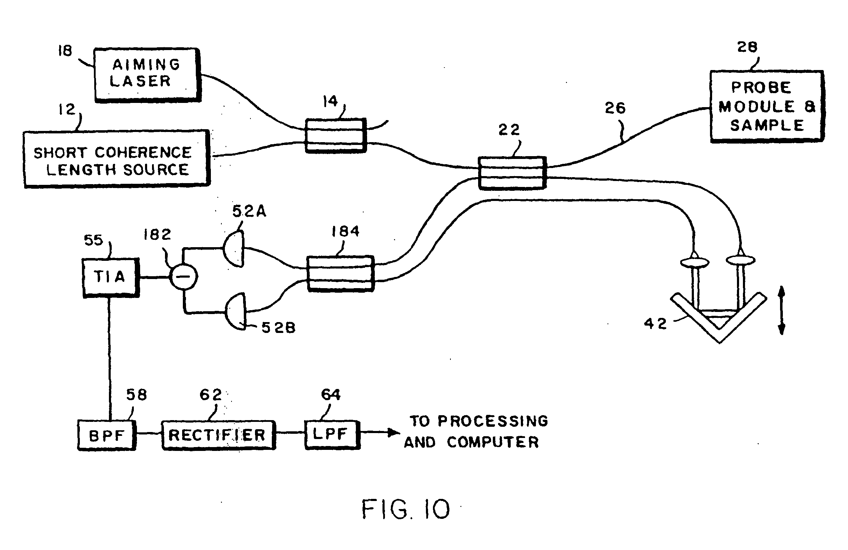

FIG. 10 Illustrates one possible balanced receiver embodiment which may be utilized where excess intensity noise is present. For this embodiment, two photodetectors 52A and 52B are used in a manner known in the art to eliminate excess intensity noise, this noise being cancelled in subtraction circuit 182. With this embodiment, an additional optical coupler 184 is provided which receives input from reflections off the sample and two faces of corner cube 42. Numerous other techniques known in the art for performing balanced detecting might also be utilized. The operation for the embodiment of the invention shown in FIG. 10 is otherwise the same as that described for example in conjunction with FIG. 1A.

A potential problem for embodiments having the transverse scanning patterns is that, with the high transverse scanning rates the embodiments require, the signal bandwidths being employed may be so great that signal aliasing may occur in an image. Signal aliasing involves variation in image intensity for a given image which may, for example, vary at the Doppler shift frequency ($f_D$). One way of compensating for such aliasing is to perform multiple scans on a single sample, to store the results of each scan in memory 74 and to average the values from the various scans in computer 72 to eliminate the aliasing variations. Other preferred ways of eliminating aliasing are to use one of the techniques previously described to obtain a modulation higher than the signal bandwidth.

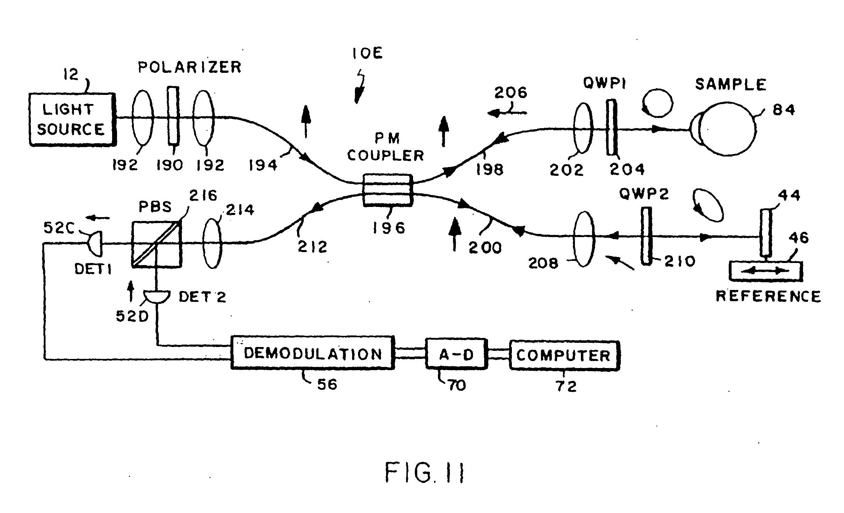

FIG. 11 illustrates the invention utilizing polarized light to detect birefringence. Light from light source 12 is polarized in a polarizer 190 sandwiched between a pair of lenses 192 before being applied to a polarization maintaining (high birefrigence) fiber 194. For purposes of illustration, polarizer 190 is shown as vertically polarizing light from source 12, vertical polarization being one of the modes of fiber 194. Fiber 194 is connected to a polarization maintaining coupler 196 which outputs the vertically polarized light on polarization maintaining fibers 198 and 200. Fiber 198 terminates in a focussing lens 202, the optical output from which is applied through a quarter wave retardation plate 204 to sample 84'. Plate 204 is preferably a zero order or low order plate which is placed and oriented in a manner so that circularly polarized light is incident on sample 84'. In the absence of sample birefringence, plate 204 converts reflected light passing therethrough to fiber 198 into horizontal polarization. In the presence of sample birefringence which causes light to travel at different speeds through the layer depending on polarization, the light reflected from sample layers which are in, or deeper than, the birefringent sample structures will in general return to the fiber in elliptical polarization states.

In the reference arm, the vertically polarized light in fiber 200 is focussed by lens 202 and a quarter-wavelength retardation plate 210 to mirror 44. Plate 210, which is also preferably zero order or low order, is oriented in such a manner that light applied to the mirror is elliptically polarized and reflections from the mirror which are returned to fiber 200 are in a linear polarization state with equal horizontal and vertical components. The sample and reference reflections are recombined with interferometric fringes in coupler 196 and applied to a polarization maintaining fiber 212. Fiber 212 terminates in a lens 214 leading to a polarizing beam splitter 216, with horizontally polarized light from the beam splitter being applied to detector 52C and vertically polarized light from the beam splitter being applied to detector 52D. Lens 214 and polarizing beam splitter 216 may be replaced by a fiber polarizing beam splitter.

The interferometric signals detected by the two detectors, which signals are both at the same Doppler shift frequency, are separately processed in demodulators 56 and A/D converters 70 (the separate demodulators and A/D converters being shown for simplicity as single units in FIG. 11) to produce two interferometric signals, a horizontal amplitude component I1 and a vertical amplitude component I2. These signals are applied to computer 72 and can be used therein to determine the round trip birefringent retardation $\phi$ in the sample light path

$$\phi = \arctan \left| \frac{I_2}{I_1} \right|$$

and to determine the amplitude $|I_t|$ for the sample reflection.

$$|I_t| = |I_1|^2 + |I_2|^2$$

Thus, by measuring the relative amplitude and phase of the two detector outputs, information on the relative phase retardation along the sample principal axes are obtained as a function of sample depth.

Birefringence is observed in structures in the eye such as the nerve fiber layer of the retina, as well as in other highly ordered biological tissues, crystals and other structures. Changes in eye nerve fiber layer thickness of 10-20 micrometers may be significant interval changes in glaucoma, and may presage the development of optic nerve head cupping and other visual field loss. Prior art techniques for measuring retinal thickness have only had a resolution on the order of 40 micrometers. However, the apparatus shown in FIG. 11 can detect the thickness of the birefringent retinal nerve fiber layer with a resolution of 10 microns. Back scattering from inside the retinal nerve fiber layer (RNFL) can be identified because the refringent retardation of back scattering from inside the RNFL increases, as for other birefringent surfaces, with depth. The range of depth over which the birefringent retardation is changing is the thickness of the RNFL and the rate of change of birefringent retardation (total retardation divided by the thickness of the RNFL) can provide a measure of the nerve axon density inside the RNFL. The back scattering and reflections from layers deeper than the RNFL will acquire a constant amount of birefringent retardation.

The ability to make such nerve fiber layer measurements provides a marked advantage in the early detection of glaucoma and in the objective assessment of progression of glaucomatous damage. Thus, weak back scattering signals from retinal substructures could be measured and could yield direct measurements of not only the overall retinal thickness, but the thickness of component sublayers as well.

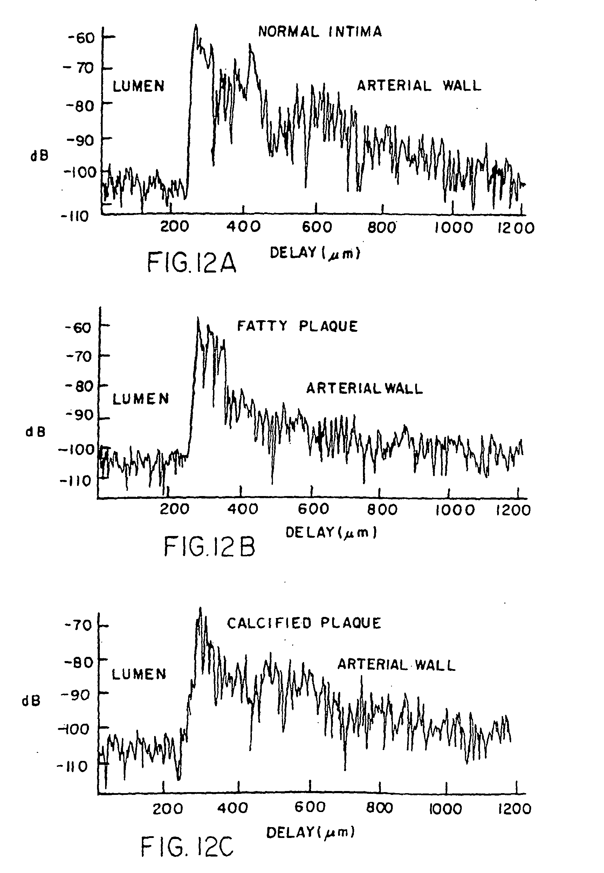

Back scattered light can also be detected from the first few millimeters of turbid tissue samples such as arterial plaque and normal arterial wall. FIGS. 12A-12C are illustrations of back scattering patterns obtained from an arterial wall which is normal and which has various types of plaque deposited thereon. The log rate of attenuation for back scatter is also different for fatty plaque than for arterial wall, providing an additional way of distinguishing plaque. A fiber optic probe of the type shown in FIGS. 5-7 could be delivered by use of an endoscope to a desired site to provide high resolution images for use in laser angioplasty and lithotripsy. This would enhance the usability of such procedures by reducing the dangers of unintentional vessel damage and rupture. This is because not only can this technique provide finer resolution than is obtainable with prior ultrasonic techniques, but also provides the ability to distinguish between arterial plaque and normal artery wall in a number of ways, including measuring birefringence and spectral properties. The internal elastic lamina of arteries are highly birefringent, which plaques are not. Plaques also have other different spectral characteristics. Such differentiation is not easily obtained with ultrasonic techniques.

Further, while specific fiber optic and bulk optic implementations have been shown, it is apparent that this invention could also be practiced utilizing other optical implementations and that other modifications in the specific equipment shown for performing the functions might be possible, depending on application. Thus, while the invention has been particularly shown and described above with reference to preferred embodiments, the foregoing and other changes in form and detail may be made therein by one skilled in the art without departing from the scope of the invention as claimed.

**Claims**

1. Apparatus for interferometrically imaging or measuring of the internal structure of a sample comprising:

    - a two beam interferometer having a reference beam path (30) with a reference reflector (44) and a measuring beam path (26) leading to the sample (84),
    - an optical radiation source (12, 12a, 12b, 79) providing light to the two beam interferometer (30, 26, 44),
    - a probe module (28) arranged in said measuring beam path (26) at its terminating portion, the probe module (28) comprising means (86, 100, 110, 95) for scanning the sample (84) by steering the direction of light propagation applied to the sample (84),
    - means (22) for combining light reflected at said reference reflector (44) and light reflected within the sample (84),
    - a detector (52, 52') detecting the superimposed light,
    - means (46, 46') for changing the longitudinal depth within said sample (84) for which the light reflected within the sample (84) interferes with light reflected at the reference reflector (44) and
    - means for processing the output signal of said detector (52, 52') to generate a longitudinally resolved image or measurement of said sample (84) including information received from reflections or scatterings in various depths within said sample (84),
    - wherein the optical radiation source (12, 12a, 12b) is a short coherence length optical source,
    - wherein the means (46) for changing the longitudinal depth are designed to change the relative length of the reference beam path (30) and the measuring beam path (26) in accordance with a predetermined velocity profile having an instantaneous velocity V at each point on the profile, wherein interference fringes occur at length matched points of the reference and measurement beam paths (26, 30), wherein the output signal has an instantaneous modulating frequency and wherein said modulating frequency includes a Doppler shift frequency at a frequency of $f_D = NV/\lambda$, where $\lambda$ is the wavelength of the radiation source,
    - wherein the apparatus further comprises

        • means for polarizing the optical radiation from the source in a selected first direction,
        • means for altering the polarization of the radiation differently for radiation applied to the reflector and to the sample, said means for altering causing reflected radiation from the reflector to be polarized in a selected second direction and causing reflected radiation from the sample to be polarized in a direction dependent on birefringence of the sample, the polarized reflected radiation from the reflector and sample being interferometrically combined,
        • means (216; 52C, 52D) for splitting and detecting the interferometrically combined output as two outputs having orthogonal polarization states, means (56) for separately processing the two outputs to obtain separate interferometric signals and means (72) for combining said interferometric signals to provide a selected indication of a birefringent profile of the sample.

2. The Apparatus of claim 1, wherein there is a bandwidth requirement to compensate for predominant low frequency noise, wherein the velocity V is not sufficient to result in a Doppler shift frequency $f_D$ which is sufficient to meet the bandwidths requirement, wherein the means (46) for changing the longitudinal depths includes means (34) for causing an additional modulation at a frequency $f_M$ for at least one of the reference beam path (30) or the measuring beam path (26) and wherein the means for processing the output signal includes a demodulator (56) which demodulates for a modulating frequency which is a selected combination of $f_D$ and $f_M$.

3. The apparatus of one of the claims 1 or 2, wherein the means for scanning the sample (84) includes at least one moveable mirror (100, 106) for steering the radiation at an angle dependent on the mirror position.

4. The apparatus of one of the claims 1 or 2, including a mirror (92) and means (95) for rotating the mirror (92) for changing its pitch to effect circular scanning.

5. The apparatus of one of the claims 1 or 2, wherein the probe module (28) is a mechanism (92, 100) for scanning internal channels (144).

6. The apparatus of one of the claims 1-5, wherein the means (46, 46') for changing the longitudinal depth includes means for periodically altering the length of the reference beam path (30), resulting in periodic changes in the depths position in the sample (84) of a length matched point of the measuring beam path (26), and further comprising a probe module (28) including means (90) for controlling the depths of focus in the sample (84) so that the depth of focus is maintained substantially at said length matched point as said point is periodically changed.

7. The apparatus of one of the claims 1-6, wherein the rates at which said means (46) for changing the longitudinal depth and said transverse scanning means are moved are such that points at all longitudinal ranges of interest are scanned for a given transverse position on the sample before the transverse scanning means causes the probe module (28) to initiate scanning at a new transverse position.

8. The apparatus of one of the claims 1-6, wherein the rates at which said means (46) for scanning the longitudinal depth and said transverse scanning means (86, 100, 110, 95) are moved are such that points at all transverse positions to be scanned in at least one transversal dimension are scanned at a given longitudinal depth in the sample (84) before the longitudinal depth changing means (46) causes scanning at a new longitudinal depth to be performed.

9. The apparatus of one of the claims 1-6, wherein said transverse scanning means (86, 100, 110, 95) includes means for performing a two dimensional transverse scan at a longitudinal depth in the sample determined by said longitudinal depth changing means (46).

10. The apparatus of claim 1, including a plurality of the apparatus of claim 1, including a plurality of measuring and reference beam paths, an optical radiation source (12a to 12c) at the proximal end of each of the plurality of paths, a reference reflector (44) at the distal end of each reference beam path and a transverse point on the sample (84) at the distal end of each measuring beam path, and wherein said means for processing (180) includes means for processing the received image from a plurality of paths to effect parallel scanning of the sample (84).

11. Apparatus of claim 1, comprising means (56) for demodulating the output of the detector (52), and means (72) for processing the demodulated output, wherein the demodulating means (56) includes filter means (58) for controlling the frequency band about said modulating frequency which is accepted by the means for demodulating (56) and comprising means for expanding the frequency band about said modulating frequency which is accepted.

12. The apparatus of claim 1, wherein the radiation source (12A, 12B) comprises means for providing short coherence length optical radiation at at least two different wavelengths $\lambda_1$, $\lambda_2$, wherein at least a first combined optical output modulated at a frequency $f_1$ and a second optical output modulated at a frequency $f_2$ is generated, and wherein means (56A, 56B) for separately demoldulating the first and the second outputs are provided.

13. Apparatus for interferometrically imaging or measuring of the internal structure of a sample comprising:

- a two beam interferometer having a reference beam path (30) with a reference reflector (44) and a measuring beam path (26) leading to the sample (84),
- a frequency modulated spectrally coherent optical radiation source (12, 12a, 12b, 79) providing light to the two beam interferometer (30, 26, 44),
- a probe module (28) arranged in said measuring beam path (26) at its terminating portion, which probe module (28) comprises means for scanning the sample by steering the direction of light propagation applied to the sample (84),
- means for changing the longitudinal depth within said sample (84) for which the light reflected within the sample (84) interferes with light reflected at the reference reflector (44) which means for changing the longitudinal depth include means (78) for modulating the frequency of the source (79) output with interference resulting in a signal having a frequency proportional to the difference between the path lengths of the measuring and the reference beam paths (26, 30).
- means (22) for combining light reflected at said reference reflector (44) and light reflected within the sample (84),
- a detector (52, 52') detecting the superimposed light, and
- means for processing the output signal of said detector (52, 52') which means generate a longitudinally resolved image of the internal structure of said sample (84) which image includes information obtained from reflected or scattered radiation received from various depths within said sample (84).

**Patentansprüche**

1. Vorrichtung zur interferometrischen Abbildung oder Messung der inneren Struktur einer Probe, wobei die Vorrichtung aufweist:

- ein zweistrahliges Interferometer, welches einen Referenzstrahlengang (30) mit einem Referenzreflektor (44) sowie einen zur Probe (84) führenden Meßstrahlengang (26) hat,
- eine optische Strahlenquelle (12, 12a, 12b, 79), die den zweistrahligen Interferometer (30, 26, 44) Licht bereit stellt,
- ein im Meßstrahlengang (26) an dessen Endabschnitt angeordnetes Meßkopfmodul (28), welches Mittel (86, 100, 110, 95) zum Scannen der Probe (34) durch Steuern der Richtung der auf die Probe (84) gerichteten Lichtausbreitung umfaßt,
- Mittel (22) zum Kombinieren von am Reflektor (44) reflektiertem Licht mit von innerhalb der Probe (84) reflektiertem Licht,
- einen Detektor (52, 52') zum Detektieren des überlagerten Lichtes,
- Mittel (46, 46') zum Ändern der in Längsrichtung gesehenen Tiefe innerhalb der Probe (84), bei der das von der Probe (84) reflektierte Licht mit am Referenzreflektor (44) reflektiertem Licht interferiert, sowie
- Mittel zum Verarbeiten des Ausgangssignals des Detektors (52, 52') zum Erzeugen eines in Längsrichtung aufgelösten Bildes oder Messung der Probe einschließlich Informationen, die aus Reflektionen oder Streuungen in verschiedenen Tiefen in der Probe erhalten sind,
- wobei die optische Strahlenquelle (12, 12b) eine optische Quelle mit kurzer Kohärenzlänge ist,
- wobei die Mittel (46) zum Ändern der in Längsrichtung gesehenen Tiefe ausgebildet sind, zum Ändern der relativen Länge des Referenzstrahlenganges (30) und des Meßstrahlenganges (26) gemäß einem vorgegebenen Geschwindigkeitsprofil, das an jedem Punkt eine Momentangeschwindigkeit V hat, wobei bei längenabgeglichenen Punkten des Referenz- und des Meßstrahlenganges (26, 30) Interferenzstreifen auftreten und das Ausgangssignal eine Momentanmodulationsfrequenz aufweist, welche eine Doppler-Verschiebungsfrequenz bei einer Frequenz $f_D = NV/\lambda$ enthält, wobei $\lambda$ die Wellenlänge der Strahlenquelle ist,
- wobei die Vorrichtung weiter aufweist:

  • Mittel zum Polarisieren der optischen Strahlung von der Quelle in einer ersten Richtung,
  • Mittel zum Verändern des Polarisationszustandes der Strahlung, welche dem Reflektor zugeführt wird, anders als der Strahlung, welche der Probe zugeführt wird, wobei die Mittel zum Verändern bewirken, daß vom Reflektor reflektierte Strahlungen in einer gewählten zweiten Richtung polarisiert ist und daß von der Probe reflektierte Strahlung in einer von Proben-Doppelbrechung abhängenden Richtung polarisiert ist, wobei die polarisierten und vom Reflektor und der Probe reflektierten Strahlungen interferometrisch kombiniert werden,
  • Mittel (216; 52C, 52D) zum Aufteilen und Detektieren der interferometrisch kombinierten Ausgänge in zwei Ausgänge mit orthogonalem Polarisationszustand, Mittel (56) zum getrennten Verarbeiten der beiden Ausgänge, um getrennte interferometrische Signale zu gewinnen, sowie Mittel (72) zum Zusammenführen der interferometrischen Signale, um eine Anzeige eines Doppelbrechungsprofils der Probe bereit zu stellen.

2. Vorrichtung nach Anspruch 1, wobei eine Bandbreitenanforderung zur Kompensation vorherrschenden Niederfrequenzrauschens besteht, wobei die Geschwindigkeit V nicht ausreicht, um eine die Bandbreitenanforderung erfüllende Doppler-Verschiebungsfrequenz $f_D$ zu erzielen, wobei die Mittel (46) zur Veränderung der in Längsrichtung gesehenen Tiefe Mittel (34) umfassen, welche für den Referenzstrahlengang (30) und/oder den Meßstrahlengang (26) eine zusätzliche Modulation bei einer Frequenz $f_M$ bewirken, und wobei die Mittel zur Verarbeitung des Ausgangssignals einen Demodulator (56) umfassen, der bei einer Modulationsfrequenz demoduliert, welche eine ausgewählte Kombination aus $f_D$ und $f_M$ darstellt.

3. Vorrichtung nach Anspruch 1 oder 2, wobei die Mittel zum Scannen der Probe (84) zumindest einen beweglichen Spiegel (100, 106) zum Steuern der Strahlung in einen von der Spiegelposition anhängigen Winkel enthält.

4. Vorrichtung nach Anspruch 1 oder 2, welche einen Spiegel (92) und Mittel (95) zum Drehen des Spiegels (92) umfaßt, um dessen Neigung zu ändern und so ein kreisförmiges Scannen zu bewirken.

**5.** Vorrichtung nach Anspruch 1 oder 2, wobei das Meßkopfmodul (28) ein Mechanismus (92, 100) zum Scannen innenliegender Kanäle (144) ist.

**6.** Vorrichtung nach einem der Ansprüche 1 bis 5, wobei die Mittel (46, 46') zum Ändern in Längsrichtung gesehenen Tiefe Mittel zum periodischen Ändern der Länge des Referenzstrahlenganges (30) umfassen, wodurch sich innerhalb der Probe (84) die Tiefenposition eines längenabgeglichenen Punktes des Meßstrahlengangs (26) periodisch ändert, und weiter ein Meßkopfmodul (28) vorgesehen ist, welches Mittel (90) zur Steuerung der Tiefe eines Fokus in der Probe (84) enthält, so daß die Tiefe des Fokus im wesentlichen an dem erwähnten, längenabgeglichenen Punkt erhalten bleibt, wenn dieser Punkt periodisch geändert wird.

**7.** Vorrichtung nach einem der Ansprüche 1 bis 6, wobei Geschwindigkeiten, mit der die Mittel (46) zum Ändern der in Längsrichtung gesehenen Tiefe sowie die transversalen Scanmittel gestellt werden, so sind, daß Punkte in allen interessierenden Längsbereichen an einer gegebenen transversalen Position an der Probe gescannt werden, bevor die transversalen Scanmittel das Meßkopfmodul (28) veranlassen, einen Scan in einer neuen transversalen Position einzuleiten.

**8.** Vorrichtung nach einem der Ansprüche 1 bis 6, wobei Geschwindigkeiten, mit der die Mittel (46) zum Scannen der in Längsrichtung gesehenen Tiefe sowie die transversalen Scanmittel (86, 100, 110, 95) gestellt werden, so sind, daß die zumindest in einer transversalen Dimension zu scannenden Punkte in einer gegebenen, in Längsrichtung gesehenen Tiefe der Probe (84) an allen transversalen Positionen gescannt werden, bevor die Mittel (46) zum Scannen der in Längsrichtung gesehenen Tiefe einen Scan bei einer neuen, in Längsrichtung gesehenen Tiefe veranlassen.

**9.** Vorrichtung nach einem der Ansprüche 1 bis 6, wobei die transversalen Scanmittel (86, 100, 110, 95) Mittel zur Durchführung eines zweidimensionalen transversalen Scans in einer in Längsrichtung gesehenen Tiefe in der Probe umfassen, welche von den Mitteln (46) zum Ändern der in Längsrichtung gesehenen Tiefe bestimmt ist.

**10.** Vorrichtung nach Anspruch 1, welche mehrere Vorrichtungen nach Anspruch 1 umfassen, mit mehreren Meß- und Referenzstrahlengängen, an deren proximalen Enden sich optische Strahlenquellen (12a bis 12c) befinden, einem Referenzreflektor (44) am distalen Ende jedes Referenzstrahlenganges und einem transversalen Punkt auf der Probe (84) am distalen Ende jedes Meßstrahlenganges, wobei die Verarbeitungsmittel (180) Mittel zur Verarbeitung des aus verschiedenen Strahlengängen empfangenen Bildes zum Bewirken eines parallelen Scannen der Probe (84) umfassen.

**11.** Vorrichtung nach Anspruch 1, die Mittel (56) zum Demodulieren des Ausgangs des Detektors (52) sowie Mittel (72) zum Verarbeiten des demodulierten Ausgangs aufweist, wobei die Demoduliermittel (56) Filtermittel (58) zum Steuern des Frequenzbandes um die von den Demodulationsmitteln (56) akzeptierte Modulationsfrequenz herum und Mittel zum Erweitern des Frequenzbandes um die akzeptierte Modulationsfrequenz herum umfassen.

**12.** Vorrichtung nach Anspruch 1, wobei die Strahlenquelle (12a, 12b) Mittel aufweist, welche optische Strahlung mit kurzer Kohärenzlänge und bei mindestens zwei verschiedenen Wellenlänge $\lambda 1$, $\lambda 2$ bereitstellen, wobei zumindest ein bei einer Frequenz $f_1$ modulierter erster kombinierter optischer Ausgang sowie ein bei einer Frequenz $f_2$ modulierter zweiter optischer Ausgang erzeugt werden, und wobei Mittel (56a, 56b) zum getrennten Demodulieren des ersten und des zweiten Ausgangs vorgesehen sind.

**13.** Vorrichtung zur interferometrischen Abbildung oder Messung der inneren Struktur einer Probe, wobei die Vorrichtung aufweist:

   - ein zweistrahliges Interferometer, welches einen Referenzstrahlengang (30) mit einem Referenzreflektor (44) sowie einen zur Probe (84) führenden Meßstrahlengang (26) hat,
   - eine frequenzmodulierte, spektral kohärente optische Strahlenquelle (12, 12a, 12b, 79), die dem zweistrahligen Interferometer (30, 26, 44) Licht bereit stellt,
   - ein im Meßstrahlengang (26) an dessen Endabschnitt angeordnetes Meßkopfmodul (28), welches Mittel zum Scannen der Probe durch Steuern der Richtung der auf die Probe (84) gerichteten Lichtausbreitung umfaßt,
   - Mittel zum Ändern der in Längsrichtung gesehenen Tiefe innerhalb der Probe (84), bei der das von der Probe (84) reflektierte Licht mit am Referenzreflektor reflektiertem Licht interferiert, wobei die Mittel zum Ändern der in Längsrichtung gesehenen Tiefe Mittel (78) zum Modulieren der Frequenz des Quellenausgang (79) umfaßt, wobei Interferenz ein Signal ergibt, dessen Frequenz proportional zum Weglängenunterschied zwischen Meß- und Referenzstrahlengang (26, 30) ist,
   - Mittel zum Kombinieren von am Referenzreflektor (44) reflektiertem Licht mit innerhalb der Probe (84) reflektiertem Licht,
   - einen Detektor (52, 52') zum Detektieren des überlagerten Lichts und

- Mittel zum Verarbeiten des Ausgangssignals des Detektors (52, 52'), wobei diese Mittel ein in Längsrichtung aufgelöstes Bild der inneren Struktur der Probe (84) erzeugen und das Bild Informationen umfaßt, die aus reflektierter oder gestreuter Strahlung erhalten sind, welche aus verschiedenen Tiefen in der Probe (84) empfangen wurde.

## Revendications

1. Appareil pour la formation interférométrique d'images ou de mesures de la structure interne d'un échantillon comprenant: .

    - un interféromètre à deux faisceaux pourvu d'une voie de faisceau de référence (30) avec un réflecteur de référence (44) et d'une voie de faisceau de mesure (26) conduisant à l'échantillon (84),
    - une source de rayonnement optique (12, 12a, 12b, 79) fournissant de la lumière à l'interféromètre à deux faisceaux (30, 26, 44),
    - un module de sonde (28) placé à sa partie d'extrémité dans ladite voie de faisceau de mesure (26), le module de sonde (28) comprenant des moyens (86, 100, 110, 95) pour explorer l'échantillon (84) en guidant la direction de propagation de lumière appliquée à l'échantillon (84),
    - des moyens (22) pour combiner la lumière réfléchie audit réflecteur de référence (44) et la lumière réfléchie dans l'échantillon (84),
    - un détecteur (52, 52') détectant la lumière superposée,
    - des moyens (46, 46') pour modifier la profondeur longitudinale dans ledit échantillon (84) pour lesquels la lumière réfléchie dans celui-ci (84) interfère avec la lumière réfléchie sur le réflecteur de référence (44) et
    - des moyens pour traiter le signal de sortie dudit détecteur (52, 52') afin de générer une image à résolution longitudinale ou des mesures dudit échantillon (84), comprenant l'information reçue des réflexions ou diffusions dans les diverses profondeurs dudit échantillon (84),
    - dans lequel la source de rayonnement optique (12, 12a, 12b) est une source optique à courte cohérence,
    - dans lequel des moyens (46) pour modifier la profondeur longitudinale sont prévus pour changer la longueur relative de la voie à faisceau de référence (30) et de la voie de faisceau de mesure (26) conformément à un profil de vitesse prédéterminée ayant la vitesse V instantanée à chaque point sur le profil, dans lequel les franges d'interférence surviennent à des points de longueur concordants des voies de faisceau de référence et de mesure (26,30) et dans lequel le signal de sortie a une fréquence de modulation instantanée, et dans lequel ladite fréquence de modulation renferme une fréquence de déplacement Doppler à la fréquence de $f_D = NV/\lambda$, où $\lambda$ représente les longueurs d'onde de la source de rayonnement,

dans lequel l'appareil comprend en outre :

    - des moyens pour polariser le rayonnement optique provenant d'une source dans une première direction choisie,
    - des moyens pour modifier la polarisation différemment pour le rayonnement appliqué au réflecteur et à l'échantillon, lesdits moyens pour modifier faisant que le rayonnement réfléchi par le réflecteur peut être polarisé dans une direction dépendant de la biréfringence de l'échantillon, les rayonnements polarisés réfléchis par le réflecteur et l'échantillon étant combinés interférométriquement,
    - des moyens (216; 52C, 52 D) pour diviser et détecter la sortie combinée interférométriquement comme deux sorties ayant des états de polarisations orthogonaux, des moyens (56) pour traiter séparément les deux sorties pour obtenir des signaux interférométriques séparés et des moyens (72) pour combiner lesdits signaux interférométriques pour obtenir une indication sélectionnée sur un profil biréfringent de l'échantillon.

2. L'appareil selon la revendication 1, dans lequel il y a une exigence de largeur de bande afin de compenser le bruit basse fréquence prédominant, dans lequel la vitesse V n'est pas suffisante pour aboutir en une fréquence $f_D$ de décalage Doppler qui soit suffisante pour satisfaire l'exigence de largeurs de bande, dans lequel les moyens (46) pour changer les profondeurs longitudinales comportent des moyens (34) pour générer une modulation supplémentaire à la fréquence $f_M$ pour au moins l'une de la voie de faisceaux de référence (30) ou la voie de faisceaux de mesure (26) et dans lequel les moyens pour traiter le signal de sortie comportent un démodulateur (56) qui démodule en une fréquence de modulation qui est une combinaison sélectionnée de $f_D$ et $f_M$.

3. L'appareil selon l'une des revendications 1 ou 2, dans lequel les moyens pour explorer l'échantillon (84) renferment au moins un miroir mobile (100, 106) pour guider le rayonnement à un angle dépendant de la position du miroir.

4. L'appareil selon l'une des revendications 1 ou 2, comprenant un miroir (92) et des moyens (95) pour mettre le miroir (92) en rotation afin de changer son pas pour le balayage circulaire.

5. L'appareil selon l'une des revendications 1 ou 2, dans lequel le module de sonde (28) est un mécanisme (92, 100) pour explorer les canaux internes (144).

6. L'appareil selon l'une des revendications 1-5, dans lequel les moyens (46, 46') pour modifier la profondeur longitudinale comportent des moyens pour modifier périodiquement la longueur de la voie de faisceau de référence (30), ce qui mène à des changements périodiques de la position de profondeur dans l'échantillon (84), d'un point de longueur concordante de la voie du faisceau de mesure (26) et comportant en outre un module de sonde (28) renfermant des moyens (90) pour commander les profondeurs de foyer dans l'échantillon (84), de sorte que la profondeur de foyer soit essentiellement maintenue audit point de longueur concordante lorsque ledit point se trouve modifié périodiquement.

7. L'appareil selon l'une des revendications 1-6, dans lequel les vitesses auxquelles lesdits moyens (46) pour modifier la profondeur longitudinale et lesdits moyens d'exploration transversale sont déplacés sont telles que des points à toutes les étendues longitudinales d'intérêt soient explorés pour une position transversale donnée sur l'échantillon avant que les moyens d'exploration transversale n'amènent le module de sonde (28) à initier l'exploration à une nouvelle position transversale.

8. L'appareil selon l'une des revendications 1-6, dans lequel les vitesses auxquelles lesdits moyens (46) pour explorer la profondeur longitudinale et lesdits moyens d'exploration transversale (86, 100, 110, 95) sont déplacés sont telles que des points à toutes les positions transversales à explorer dans au moins une dimension transversale soient explorés à une profondeur longitudinale donnée dans l'échantillon (84), avant que les moyens pour modification de la profondeur longitudinale (46) n'entraînent l'exploration à une nouvelle profondeur longitudinale.

9. L'appareil selon l'une des revendications 1-6, dans lequel lesdits moyens d'exploration transversale (86, 100, 110, 95) comprennent des moyens pour réaliser une exploration transversale à deux dimensions à une profondeur longitudinale de l'échantillon déterminée par lesdits moyens pour modification de la profondeur longitudinale (46).

10. L'appareil selon la revendication 1, comportant une pluralité d'appareils selon la revendication 1, comportant une pluralité de voies à faisceau de mesure et de référence, une source de rayonnement optique (12a à 12c) à l'extrémité proximale de chacune de la pluralité de voies, un réflecteur de référence (44) à l'extrémité distale de chaque voie à faisceau de référence et un point transversal sur l'échantillon (84) à l'extrémité distale de chaque voie de faisceau de mesure, et dans lequel ledit moyen de traitement (1 80) comprend des moyens pour traiter l'image reçue d'une pluralité de voies afin d'effectuer une exploration parallèle de l'échantillon (84).

11. L'appareil selon la revendication 1, comprenant des moyens (56) pour démoduler la sortie du détecteur (52) et des moyens (72) pour traiter la sortie démodulés, des moyens de démodulation (56) comprenant des moyens à filtre (58) pour commander la bande de fréquence par rapport à ladite fréquence de modulation qui est acceptée par les moyens de démodulation (56), et comprenant des moyens d'élargissement de la bande de fréquence de ladite fréquence de modulation acceptée.

12. L'appareil selon revendication 1, dans lequel la source de rayonnement (12A, 12B) comprend des moyens fournissant un rayonnement optique à cohérence courte à au moins deux différentes longueurs d'onde, $\lambda_1$, $\lambda_2$, dans lequel sont générés au moins une première sortie optique combinée, modulée à la fréquence $f_1$, et une seconde sortie optique, modulée à la fréquence $f_2$, et dans lequel sont prévus des moyens (56A, 56B) pour démoduler séparément les première et seconde sorties.

13. Appareil pour la formation interférométrique d'images ou de mesures de la structure interne d'un échantillon comprenant :

- un interféromètre à deux faisceaux pourvu d'une voie de faisceau de référence (30) avec un réflecteur de référence (44) et d'une voie de faisceau de mesure (26) conduisant à l'échantillon (84),
- une source de rayonnement optique spectralement cohérent, modulé en fréquence (12, 12a, 12b, 79), fournissant la lumière aux deux interféromètres à faisceau (26),
- un module de sonde (28) placé dans la partie d'extrémité de ladite voie du faisceau de mesure (26), le module de sonde (28) comprenant des moyens pour explorer l'échantillon en guidant la direction de propagation de la lumière appliquée à l'échantillon,
- des moyens pour modifier la profondeur longitudinale dans ledit échantillon (84) pour lesquels la lumière réfléchie dans l'échantillon (84) interfère avec la lumière réfléchie sur le réflecteur de référence (44), lesdits moyens pour modifier la profondeur longitudinale comportant des moyens (78) pour moduler la fréquence de la sortie de la source (79) avec des interférences fournissant un signal ayant une fréquence proportionnelle à la différence entre la longueur de la voie du faisceau de mesure et celle du faisceau de référence (26, 30),

- des moyens (22) pour combiner la lumière réfléchie audit réflecteur de référence (44) et la lumière réfléchie dans l'échantillon,

- un détecteur (52, 52') détectant la lumière superposée et

- des moyens pour traiter le signal de sortie dudit détecteur (52, 52') lesdits moyens générant une image de la structure interne dudit échantillon (84) à résolution longitudinale, ladite image incluant l'information obtenue par le rayonnement réfléchi ou diffusé reçu de différentes profondeurs à l'intérieur dudit échantillon (84).

FIG. 1A

EP 0 581 871 B2

18
AIMING
LASER

10B

28
PROBE
MODULE 8
SAMPLE

SIGNAL
ENERATOR

20

14

79
COHERENT FM
MODULATED
OPTICAL
SOURCE

22

26

78

50

30

52'

54

55'
TIA

44

REFERENCE
MIRROR

81
SIGNAL
PROCESSOR

70
A/D

72
COMPUTER

FIG. I B

FIG. IC

FIG. 1D

FIG. 2A

FIG.2B

FIG 2C

PROBE
MODULE

82

84

FIBER

26

80

86

SAMPLE

FIG. 3A

90    92    100

FIBER

26    98

104

94

96

102

SAMPLE    84'

FIG. 3B

FIG.3C

FIG.3D

TRANSVERSE
SCANNING

SAMPLE

FIBER

26

103  101

107

105

84'

## FIG. 4A

TRANSVERSE
SCANNING

109

SAMPLE

FIBER

26

103  101

107

105

84

## FIG. 4B

VESSEL
WALL 120

OUTER
SHEATH 124

121

130

FIBER

26

ROTATING
SHEATH 122

126  128

## FIG. 5

FIG. 6

FIG. 7

TRANSVERSE
X

INCIDENT
BEAM

Z LONGITUDINAL
(RANGE)
(DEPTH)

FIG.8A

Y
TRANSVERSE

TRANSVERSE
X

INCIDENT
BEAM

Z LONGITUDINAL
(RANGE)
(DEPTH)

FIG.8B

Y
TRANSVERSE

FIG. 8C

FIG. 9

FIG. 10

FIG.II

FIG.12A

FIG.12B

FIG.12C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 2528209 A1
- DE 3201801 A1

- GB 2191855 A

**Non-patent literature cited in the description**

- **Huang et al.** Optical coherence tomography. *Science,* 22 November 1991, vol. 254, 1178-1181

- **Boef.** Two-wavelength scanning spot interferometer using single-frequency diode Lasers. *Applied Optics,* 15 January 1998, vol. 27 (2), 306-309